# EUROPEAN PATENT SPECIFICATION

(11) **EP 1 667 727 B1**
(45) Date of publication and mention of the grant of the patent: **09.07.2008**
(21) Application number: 04783536.8
(22) Date of filing: 09.09.2004
(51) Int. Cl.: A61K 47/48, A61K 31/132

(54) **POLYAMINE-METAL CHELATOR CONJUGATES**
POLYAMIN-METALLCHELATBILDNER-KONJUGATE
CONJUGUES POLYAMINE-CHELATEUR DE METAL

(30) Priority: 09.09.2003 US 501341 P
(43) Date of publication of application: 14.06.2006
(73) Proprietor: University of Florida Research Foundation, Inc., Gainesville, FL 32603 (US)
(72) Inventor: BERGERON, Raymond, J., Jr., Gainesville, FL 32653 (US)
(74) Representative: Stevens, Ian Edward
(86) International application number: PCT/US2004/029318
(87) International publication number: WO 2005/023310

(56) References cited:
- HNATOWICH D J ET AL: "THE PREPARATION AND LABELING OF DTPA-COUPLED ALBUMIN" INTERNATIONAL JOURNAL OF APPLIED RADIATION AND ISOTOPS, PERGAMON PRESS, NEW YORK, NY, US, vol. 33, no. 5, May 1982 (1982-05), pages 327-332, XP002002848 ISSN: 0020-708X
- SAFAVY AHMAD ET AL: "De novo synthesis of a new diethylenetriaminepentaacetic acid (DTPA) bifunctional chelating agent" BIOCONJUGATE CHEMISTRY, vol. 13, no. 2, March 2002 (2002-03), pages 317-326, XP002314458 ISSN: 1043-1802
- DELCROS JEAN-GUY ET AL: "Effect of spermine conjugation on the cytotoxicity and cellular transport of acridine." JOURNAL OF MEDICINAL CHEMISTRY, vol. 45, no. 23, 7 November 2002 (2002-11-07), pages 5098-5111, XP002314459 ISSN: 0022-2623
- WANG CHAOJIE ET AL: "Molecular requirements for targeting the polyamine transport system. Synthesis and biological evaluation of polyamine-anthracene conjugates." JOURNAL OF MEDICINAL CHEMISTRY, vol. 46, no. 13, 19 June 2003 (2003-06-19), pages 2672-2682, XP002314461 ISSN: 0022-2623
- MARTIN BENEDICTE ET AL: "N-benzylpolyamines as vectors of boron and fluorine for cancer therapy and imaging: Synthesis and biological evaluation" JOURNAL OF MEDICINAL CHEMISTRY, vol. 44, no. 22, 25 October 2001 (2001-10-25), pages 3653-3664, XP002314543 ISSN: 0022-2623
- BERGERON R J ET AL: "THE ROLE OF CHARGE IN POLYAMINE ANALOGUE RECOGNITION" JOURNAL OF MEDICINAL CHEMISTRY, AMERICAN CHEMICAL SOCIETY. WASHINGTON, US, vol. 38, no. 38, 1995, pages 2278-2285, XP001074205 ISSN: 0022-2623
- BERGERON R J ET AL: "ANTINEOPLASTIC AND ANTIHERPETIC ACTIVITY OF SPERMIDINE CATECHOLAMIDE IRON CHELATORS" BIOCHEMICAL AND BIOPHYSICAL RESEARCH COMMUNICATIONS, vol. 121, no. 3, 1984, pages 848-854, XP009042905 ISSN: 0006-291X
- KONTOGHIORGHES G J ET AL: "The design and development of deferiprone (L1) and other iron chelators for clinical use: Targeting methods and application prospects" CURRENT MEDICINAL CHEMISTRY, vol. 11, no. 16, August 2004 (2004-08), pages 2161-2183, XP009042933 ISSN: 0929-8673
- BERGERON RAYMOND J ET AL: "Polyamine-iron chelator conjugate." JOURNAL OF MEDICINAL CHEMISTRY, vol. 46, no. 25, 11 November 2003 (2003-11-11), pages 5478-5483, XP002314464 ISSN: 0022-2623
- BERGERON R J ET AL: "Methoxylation of desazadesferrithiocin analogues: Enhanced iron clearing efficiency" JOURNAL OF MEDICINAL CHEMISTRY, AMERICAN CHEMICAL SOCIETY. WASHINGTON, US, vol. 46, no. 8, 10 April 2003 (2003-04-10), pages 1470-1477, XP002265510 ISSN: 0022-2623

## Description

### GOVERNMENT SUPPORT

The invention was supported, in whole or in part, by a grant R.01-DK49108 from the National Institutes of Health. The Government has certain rights in the invention.

### BACKGROUND OF THE INVENTION

Metal ions have an array of biological functions that include charge neutralization, voltage gating, signaling, oxygen transport, electron transport, structure stabilization and regulation and contributing to enzymatic activity. However, the activity of the metal ions depends on the both the location of and the quantity of the metal. When an excess of metal is present or if a metal is present in an undesired location, this results in a condition typically referred to as metal overload.

Metal overload conditions can result from a genetic defect (e.g., excess absorption or excess accumulation of metal), diet or poisoning. Metal overload conditions also occur in subjects with certain types of anemia (e.g., anemia resulting from thalassemia) where the subject receives regular blood transfusions. The iron from these transfusions accumulates in the subject over time and eventually causes biological damage.

The metals of greatest concern in metal overload conditions are the redox-active metals, such as iron, chromium and copper. These redox-active metals can initiate free radical damage in a subject, such as through reaction with a peroxide or a thiol. The reaction of iron(II) and hydrogen peroxide is known as the Fenton reaction, which produces hydroxyl radical. Hydroxyl radical is highly reactive and is able to abstract hydrogen atoms from many organic molecules, which can lead to significant biological damage. Therefore, such metal overload conditions need to be treated.

Although metal deficiencies can generally be treated through dietary means, the treatment for metal overload conditions is more difficult. Therapy for metal overload disease often involves treating a subject with a chelating agent in order to remove the excess metal. The chelating agent has one or more functional groups that will bind to the metal. The chelating functional groups are polar and often charged, such that chelating agents are not readily taken up by cells. As a result, it can be difficult to achieve a therapeutic amount of a chelating agent within a cell and less metal is removed from the cell than desired. These factors limit the success of chelation therapy.

Metal chelators also have applications in conditions not directly related to metal overload, such as cancer. The success of treating these additional conditions with a metal chelator is also limited by the generally low concentration of chelator that is transported into cells. Thus, there is a need to find a method to increase the uptake of chelating agents into a cell in order to increase the effectiveness of chelation therapy and other treatments with metal chelators.

Wang et al (J. Med Chem., 2003, 46, 2672-2682) describes the synthesis and evaluation of a series of nine N¹-(9-anthracenylmethyl) tetraamines for cytotoxicity in L1210, α-difluoromethylornithine (DFMO)-treated L1210, Chinese hamster ovary (CHO) and CHO-MG cell lines.

Bergeron et al (Biochemical and Biophysical Research Communications, vol. 121, No. 3, 1984, pages 848-854) discloses the synthesis and biological testing of a series of iron chelating agents including the bacterial siderophores, parabactin and bis-N¹, N⁸ (2,3 dihydroxybenzoyl) spermidine, and four related compounds.

Bergeron et al (J. Med Chem., 2003, 46, 5478-5483) describes that polyamines can serve as vectors for the intracellular delivery of the bidentate chelator 1,2-dimethyl-3-hydroxypyridin-4-one (L1).

Bergeron et al (J. Med Chem., 2003, 46, 1470-1477) discloses the impact of altering the octanol-water partition properties (log P) of analogues of desazadesferrithiocin, on the ligands' iron clearing properties.

### SUMMARY OF THE INVENTION

It has now been found that the uptake of metal chelating agents can be significantly increased by reacting metal chelating agents with polyamines, thereby resulting in compounds comprising a first moiety that is the metal chelator and a second moiety that is the polyamine. In one example, 1-(12-amino-4,9-diazadodecyl)-2-methyl-3-hydroxy-4-(1*H*)-pyridinone was synthesized from spermine and 3-*O*-benzylmaltol. This compound was shown to form a 3:1 complex with iron(III) and was taken up by the polyamine transporter of L1210 leukemia cells 1900-fold against a concentration gradient. This compound is also effective as an anti-cancer agent, as it was at least 230 times more active in suppressing the growth of L1210 murine leukemia cells than the parent ligand, 1,2-dimethyl-3-hydroxypyridin-4-one. Compounds comprising spermine and a poly(hydroxamic acid) were also taken up by the polyamine transporter and were effective in suppressing the growth of leukemia cells.

The present invention relates to a compound as defined in claim 1. Such compounds are referred to in the present application as "conjugates". A further embodiment is defined in claim 2.

In one embodiment, the conjugate is represented by Structural Formula (I): 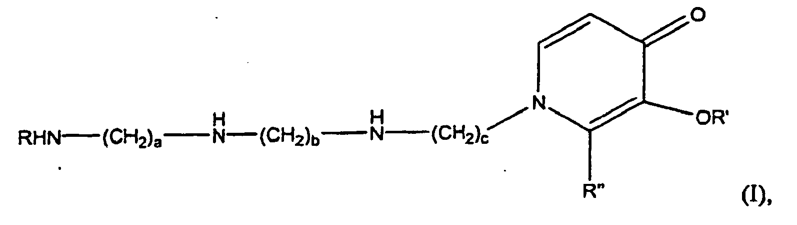 where:
a, b and c are each independently integers from 1 to 5;
R is -H or a substituted or unsubstituted alkyl, acyl or aryl group, preferably -H or a substituted or unsubstituted alkyl or aryl group;
R' is-H or a substituted or unsubstituted acyl group; and
R" is-H or a substituted or unsubstituted alkyl group,
or a salt, solvate or hydrate thereof.

In another embodiment, the conjugate is represented by Structural Formula (II): 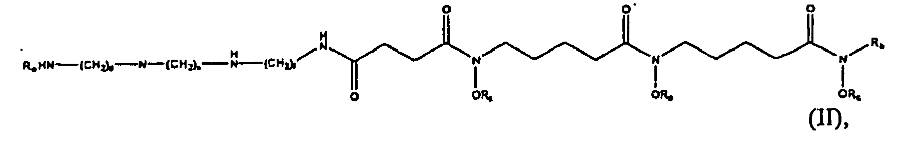 where:
d, e and f are each independently integers from I to 5;
Rₐ is -H or a substituted or unsubstituted alkyl, acyl or aryl group, preferably -H or a substituted or unsubstituted alkyl or aryl group;
R_{b} is -H or a substituted or unsubstituted alkyl group or a substituted or unsubstituted hydrocarbyl group interrupted by one or more oxygen atoms; and
each R_{c} is independently -H or a substituted or unsubstituted acyl group,
or a salt, solvate or hydrate thereof.

In yet another embodiment, the conjugate is represented by Structural Formula (III): 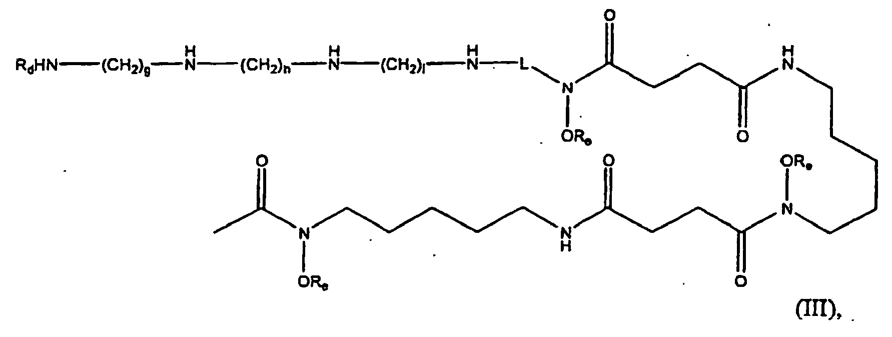 where:
g, h and i are each independently an integer from 1 to 5;
L is a linking group;
R_{d} is -H or a substituted or unsubstituted alkyl, acyl or aryl group, preferably -H or a substituted or unsubstituted alkyl or aryl group; and
each Rₑ is independently -H or a substituted or unsubstituted acyl group,
or a salt, solvate or hydrate thereof.

The present invention also includes pharmaceutical compositions comprising a pharmaceutically acceptable carrier or diluent and one of the conjugates disclosed herein.

The compounds and pharmaceutical compositions of the invention can be used in the manufacture of a medicament for treating a cancer or tumor in a subject, comprising administering the compounds or pharmaceutical compositions to the subject.

The compounds and pharmaceutical compositions of the invention can also be used in the manufacture of a medicament for treating a subject suffering from a metal overload condition, comprising administering the compounds or pharmaceutical compositions to the subject.

The present invention includes the use of a compound or pharmaceutical composition disclosed herein in medical therapy. The invention also includes the use of a compound or pharmaceutical composition disclosed herein in the manufacture of a medicament for use in treating conditions disclosed herein.

The polyamine-metal chelator conjugates of the present invention have the advantage of maintaining the metal chelating properties of the molecule, while greatly improving the uptake of the metal chelator into cells. This allows larger concentrations of a metal chelator to be present in cells. The increased intracellular concentrations of a metal chelator increase the effectiveness of the chelator as a therapeutic agent, such as in retarding the growth of malignant cells.

### BRIEF DESCRIPTION OF THE DRAWINGS

FIG. 1 shows a schematic synthesis of a spermine-L1 conjugate.
FIGS. 2A and 2B show Job's plots of the spermine-L1 conjugate and L1, respectively, with iron(III).
FIG. 3 shows the L1210 cell growth inhibition and transport for several monosubstituted polyamines, L1 and the spermine-L1 conjugate.
FIG. 4 shows the effect of several monosubstituted polyamines, L1 and the spermine-L1 conjugate on polyamine pools in L1210 cells.
FIG. 5 shows the impact of several monosubstituted polyamines, L1 and the spermine-L1 conjugate on ornithine decarboxylase (ODC), *S*-adenosylmethionine decarboxylate (AdoMet DC) and Spermine/Spermidine-N¹-Acetyltransferase (SSAT) in L1210 cells.
FIG. 6 shows the schematic synthesis of a spermine derivative of a triether amideless desferrioxamine.
FIGS. 7A-7C show the schematic synthesis of a norspermidine-desferrioxamine conjugate.
FIG. 8 shows the schematic synthesis of a protected norspermidine with a 4-hydroxybutyl connector at *N*¹.
FIG. 9 shows the schematic synthesis of a norspermidine-4-hydroxydesazadesmethyldesferrithiocin conjugate from norspermidine with a 4-hydroxybutyl connector at *N*¹.
FIG. 10 shows the Job's plot of the norspermidine-4-hydroxydesazadesmethyldesferrithiocin conjugate with iron(III).
FIGS. 11A and 11B show the IC₅₀ values of various polyamines, iron chelators and polyamine-chelator conjugates in inhibiting the growth of L1210 cells (Example 12) and show the intracellular concentration of these compounds (Example 14).
FIG. 12 shows the iron clearing efficiency of conjugated and unconjugated chelators in rodents.
FIG. 13 shows the biliary ferrokinetics of free (*S*)-4'-hydroxydesazadesferrithiocin (**8**), its free acid conjugate with norspermidine (**9**) and the corresponding ethyl ester (**10**) at a dose of 300 µmol/kg administered subcutaneously to bile duct cannulated rats (n = 4 per compound).

### DETAILED DESCRIPTION OF THE INVENTION

The present invention is directed to conjugate compounds and salts, solvates and hydrates thereof, where the conjugates comprise a metal chelator moiety and a polyamine moiety. The polyamine typically includes three or more nitrogen atoms that are capable of being positively charged at physiological pH. In a preferred embodiment, the polyamine moiety comprises three or more nitrogen atoms that are capable of being positively charged when the pH is from about 7.0 to about 7.6.

A metal chelator is defined herein as a molecule that has two or more donor atoms that can coordinate to a central metal atom, thereby forming a complex having a cyclic structure. The donor atoms can optionally be protected by a hydrolyzable functional group, such that the chelator has an overall positive or neutral charge, typically a neutral charge. It is particularly preferred that negatively-charged groups in a metal chelator are protected with a hydrolyzable functional group, thereby producing an uncharged protected chelating group.

A metal-chelator complex is thermodynamically stable, which is believed to derive from the entropy resulting from the release of non-chelating ligands (e.g., solvents, particularly water) from the coordination sphere of the metal. Chelators, as used herein, are also intended to encompass macrocycles. Accordingly, metal chelator moieties in conjugates of the invention have two or more atoms or two or more functional groups that can bind to a metal. Metal chelator moieties of the invention can be, for example, bidentate, tridentate, tetradentate, pentadentate, hexadentate, heptadentate, octadentate, nonadentate or decadentate. In one embodiment, the metal being chelated is iron(II) or iron(III) and the metal chelator moiety is pentadentate or less. In another embodiment, the metal being chelated is iron(II) or iron(III) and the metal chelator moiety is hexadentate or greater.

The metal chelator is capable of binding to one or more metals, typically a transition metal, main group metal, heavy metal, lanthanide or actinide. Aluminum and gallium are main group metals. Transition metals include scandium, titanium, vanadium, chromium, manganese, iron, cobalt, nickel, copper, zinc, yttrium, zirconium, molybdenum, palladium, silver, platinum and gold. Heavy metals include cadmium, lead, bismuth, mercury, ruthenium, rhodium, indium, tin, hafnium, tantalum, tungsten, rhenium, osmium, iridium, thallium and polonium. Lanthanides include lanthanum, cerium, praseodymium, neodymium, promethium, samarium, europium, gadolinium, terbium, dysprosium, holmium, erbium, thulium, ytterbium and lutetium. Actinides include actinium, thorium, protactinium, uranium, neptunium, plutonium, americium, curium, berkelium and californium.

The metal chelator moiety of the conjugates disclosed herein can be characterized by its formation constant with a metal ion, such as iron(II) or iron(III). For example, the metal chelator moiety can have an overall formation constant of at least about 10¹² at about 25°C, about pH 7 and about 0.1 M molar strength. Preferably, the metal chelator moiety has an overall formation constant of at least about 10²⁵ under these conditions. More preferably, the overall formation constant is at least about 10³⁵ under the stated conditions. A metal-metal chelator pair can be chosen to have an overall formation constant of at least about 10³⁰, 10⁴⁰, 10⁴⁵ or 10⁵⁰ at about 25°C, about pH 7 and about 0.1 M molar strength.

Metal chelators moieties can also be chosen based upon their selectivity for one or a few (e.g., two, three) particular metal ions. Preferably, a selective metal chelator moiety has an overall formation constant for the target metal ion of at least about an order of magnitude greater than any other metal ion. More preferably, a selective metal chelator moiety has an overall formation constant for the target metal ion at least about three orders of magnitude greater than any other metal ion.

Metal chelator moieties of the invention typically have two or more nucleophilic or negatively-charged functional groups. Suitable chelators (with appropriate functional groups) for metals can be chosen using the principles of hard-soft acid-base theory. In general, a "hard" metal ion forms more stable complexes with a "hard" base and a "soft" metal ion forms more stable complexes with a "soft" base. Hard metal ions are characterized by a high charge and/or a small ionic radius and include Mn²⁺, Al³⁺, Ga³⁺, Tl³⁺, Ln³⁺, Ti²⁺, Yb²⁺ Sm²⁺, Eu²⁺, Cr³⁺, Co³⁺ and Fe³⁺. Soft metal ions tend to have a low charge and/or a large ionic radius and include Cu⁺, Ag⁺, Au⁺, Tl⁺, Hg⁺, Cd²⁺, Pd²⁺, Pt²⁺, Pt⁴⁺ and Hg²⁺. Metal ions of intermediate hardness include Fe²⁺, Co²⁺, Ni²⁺, Cu²⁺, Zn²⁺, Pb²⁺, Sn²⁺, Tc²⁺ and Ru²⁺. Hard bases (ligands) are characterized by relatively small ionic radii and high electronegativities (e.g., do not readily donate electron density) and include hydroxyl, hydroxamate, ether, phosphate ester, amine and carboxylate groups. Soft bases have lower electronegativities and include thiols, thioethers, phosphines and isonitriles. Imidazole, aryl amines and pyridine are bases of intermediate softness.

Iron chelator moieties refer to moieties capable of chelating iron(II), iron(III) or both. Suitable iron chelator moieties contain two or more atoms or two or more functional groups that can bind to iron. Suitable functional groups include, but are not limited to, hydroxamate, catechol, porphyrin, amine, hydroxyl, carboxylate, thiol, imidazole, phenol or dithiocarbamate groups and combinations thereof. For example, an iron chelator moiety can contain more than one type of functional group.

Common iron chelators include ethylenediaminetetraacetic acid (EDTA), nitriloacetic acid (NTA), diethylenetriaminepentaacetic acid (DTPA) and N,N'-bis (2-hydroxybenzyl) ethylenediamine-N,N'-diacetic acid (HBED).

A general class of iron chelators are the siderophores, which are a diverse groups of iron chelators that are produced by microorganisms. Many of the siderophores have hydroxamate or catechol moieties. Examples of siderophores include enterobactin, parabactin, agrobactin, fluviabactin, vulnibactin, vibriobactin, ferrioxamine B, ferrioxamine E, aerobactin, and ferrichrome.

A type of siderophore is desferrithiocin and its analogues and derivatives. The structure of desferrithiocin is shown below:

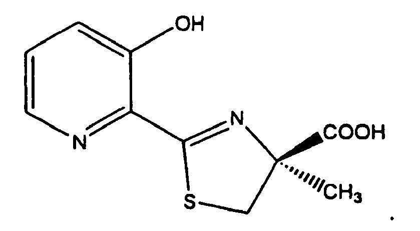

Examples of other siderophores are represented by Structural Formulas (V) and (VI): 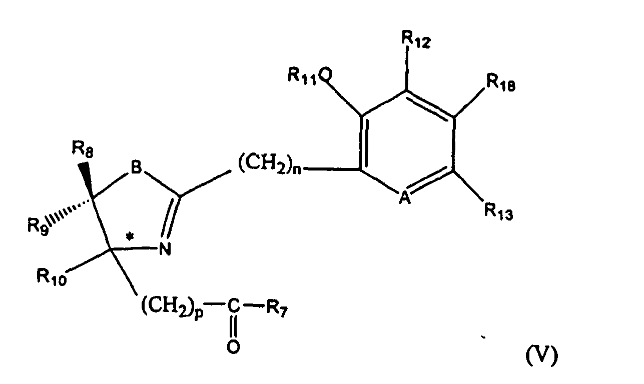 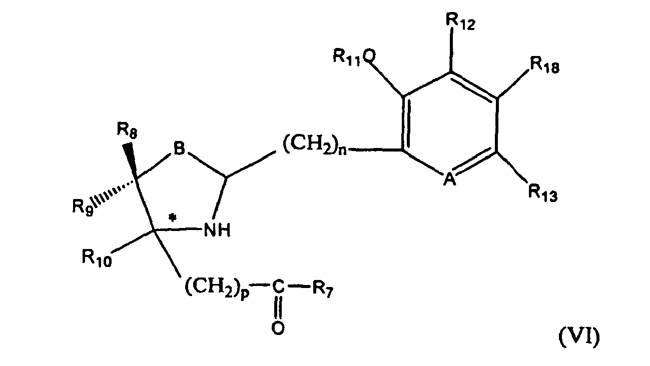 where:
R₇ is -OH, -OR₁₄, or -N(OH)R₁₅;
R₈ is -H, -CH₃ or an alkyl group of 2-6 carbons;
R₉ is -H, -CH₃ or an alkyl group of 2-6 carbons;
R₁₀ is -H, -CH₃ or an alkyl group of 2-6 carbons, or R₈ and R₉ together form a double bond;
R₁₁ is -H or acyl;
R₁₂ is -H, -OH, -O-acyl, -O-alkyl, or -L-X;
R₁₃ is -H, -OH, alkyl, a halogen, -L-Y, or is -C=C-C=C-, which, together with R₁₈, forms a fused ring system as follows: 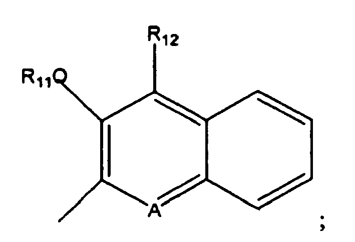
R₁₄ is alkyl, or optionally substituted benzyl;
R₁₅ is -H, alkyl, optionally substituted benzyl, 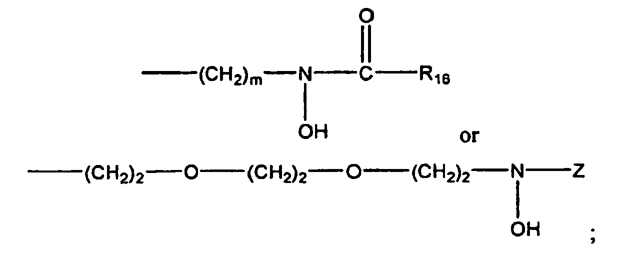
R₁₆ is -H, alkyl or optionally substituted benzyl;
R₁₈ is -H, -OH, -O-acyl, -O-alkyl or an available electron;
A is -N-, -CH- or -C(OH)-;
B is -S-, -O-, -NR₁₆-, -CH₂- or -CH₂S-;
L is an alkylene group of 3 to about 20 carbon atoms which is optionally interrupted by one or more oxygen atoms;
a is 2 or 3;
m is an integer from 1 to 8;
n is 0, 1 or 2;
p is 0, 1 or 2;
X is 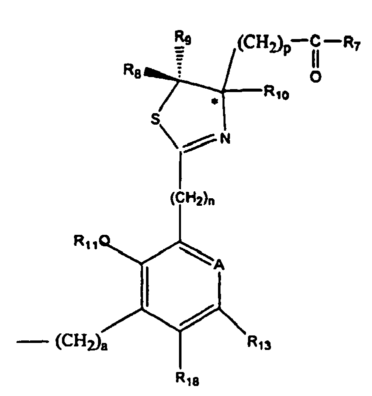 or 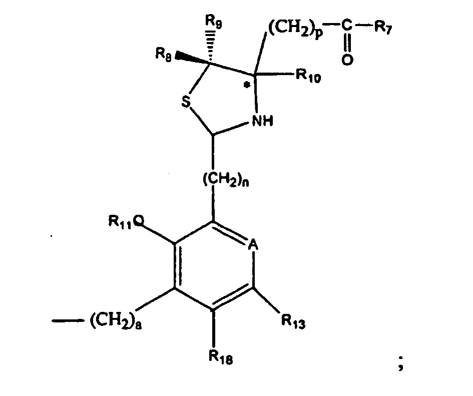
Y is 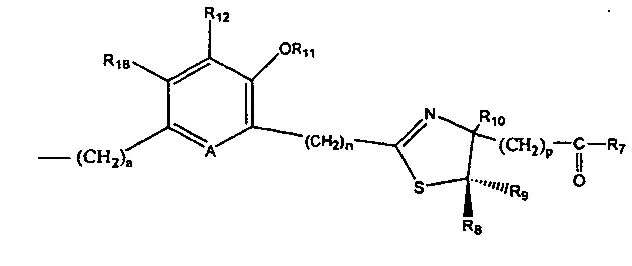 or 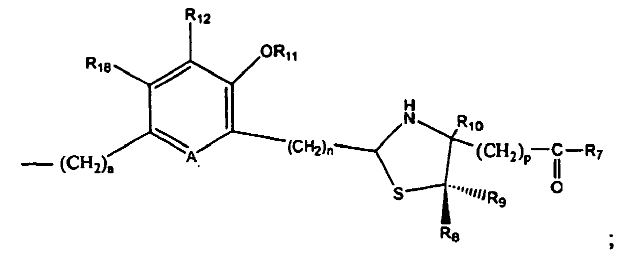 and
Z is 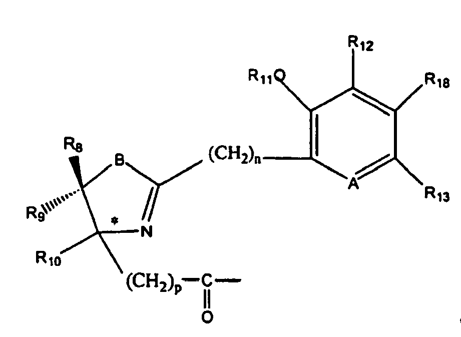 or 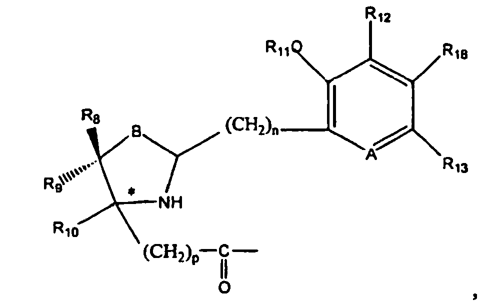
wherein each of the substituents shown is defined above, or a pharmaceutically acceptable salt of the compound represented by Structural Formula (V) or (VI) or a stereoisomer of the compound or mixture of stereoisomers.

A preferred analogue of desferrithiocin is 2-(2,4-dihydroxyphenyl)-4-(*S*)-methylthiazoline-4-carboxylic acid, also known as 4-hydroxy-desazadesferrithiocin.

Additional iron chelators are described in "Iron Chelators and Therapeutic Uses," by R.J. Bergeron, J.S. McManis, W.R. Weimar, J. Wiegand and E. Eiler-McManis in Burger's Medicinal Chemistry and Drug Discovery, Sixth Edition, Volume 3: Cardiovascular Agents and Endocrines, edited by D.J. Abraham, John Wiley & Sons, Inc., 2003 U.S. Patent Nos. 6,083,966, 6,521,652, 6,525,080 and 6.559,315, also provide iron chelators for use in the present invention. Further iron chelators are disclosed in PCT Application No. PCT/US03/28304, filed September 9, 2003.

Iron chelating moieties of the invention can be protected by a cleavable protecting group. A particularly suitable protecting group for amines and hydroxyl groups is an acyl group.

The means by which the metal chelator moiety and the polyamine moiety are attached to form the conjugate molecule is not believed to be crucial. However, it is understood that the means of attachment should not deleteriously effect the chelating ability of the metal chelator moiety or reduce the charge of the polyamine moiety to less than tricationic. The polyamine moiety is preferably attached by a terminal (primary) nitrogen, either directly or indirectly, to the metal chelator moiety, as shown diagrammatically in the structure below:

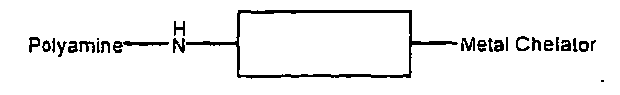

In one embodiment of the invention, the polyamine moiety and the metal chelator moiety are directly connected by a covalent bond, as shown below:
Polyamine-Metal Chelator
These conjugates are formed, for example, by directly reacting a metal chelator with a polyamine to form the conjugate, such as shown in Example 1.

In another embodiment, the polyamine moiety and the metal chelator moiety are indirectly connected by a linking group, as shown below:
Polyamine-Unking Group-Metal Chelator
These conjugates are formed, for example, by reacting either a polyamine or a metal chelator with a linking group precursor to form an intermediate product, and subsequently reacting the intermediate product with a metal chelator or a polyamine.

The polyamine moiety and the metal chelator moiety can be attached, either directly or indirectly, by a cleavable functional group. The cleavable functional group can originate with the parent polyamine, metal chelator and/or the linking group precursor.

The polyamine moiety can be derived from a polyamine that is naturally occurring. Although such naturally occurring polyamines may slow polyamine synthesis through feedback mechanisms, such polyamines should not substantially alter polyamine metabolism. Such naturally occurring polyamines include spermine, spermidine, norspermine, norspermidine, homospermine and homospermidine. Spermine, spermidine, norspermine and norspermidine occur naturally in mammalian cells. The polyamine moiety can also be derived from a polyamine that is not naturally occurring. These non-naturally occurring polyamines will generally alter polyamine metabolism, because such polyamines are not readily processed by polyamine-metabolizing enzymes (e.g., polyamine oxidase). Such non-naturally occurring polyamines include terminally alkylated polyamines such as terminally alkylated spermine, spermidine, norspermine, norspermidine, homospermine and homospermidine. A terminally alkylated polyamine is preferably monoalkylated at a terminal nitrogen atom. When an alkylated polyamine is used, it is preferred that a non-alkylated nitrogen atom of the polyamine connects the polyamine moiety to the remainder of the conjugate molecule (i.e., a primary amine).

One group of polyamines that are suitable for use in the present invention is represented by Structural Formula (IV):

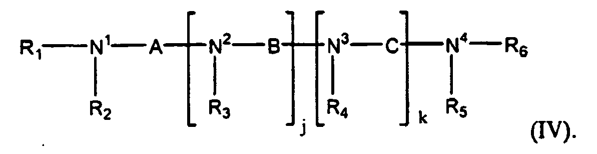

R₁ to R₆ are independently hydrogen, alkyl, aryl, aryl alkyl, cycloalkyl or any of the foregoing where the alkyl chain is interrupted by one or more etheric oxygen atoms.

N¹, N², N³ and N⁴ are nitrogen atoms capable of protonation at physiological pH's.

j and k are independently integers from 1 to 4.

A, B and C are independently bridging groups between the nitrogen atoms. The bridging groups can be alkylene groups having, for example, from 1-8 carbon atoms, cycloalkanes, cycloalkenes or heterocyclic groups that incorporate within the ring one of the nitrogen atoms of the polyamine. Preferably, the bridging groups maintain the distance between nitrogen atoms such that the polyamine is capable of uptake by a target cell upon administration to a human or non-human animal. The bridging groups can also maintain the distance between nitrogen atoms such that upon uptake by the target cell, the polyamine competitively binds via an electrostatic interaction between the positively charged nitrogen atoms to substantially the same biological counter-anions as the intracellular natural polyamines in the target cell.

Additional suitable polyamines for use in the present invention can be found in U.S. Patent Nos. 5,091,576, 5,342,945, 5,455,277, 5,866,613, 5,886,051, 6,184,232 and 6,342,534.

For conjugates of the invention represented by Structural Formula (1): 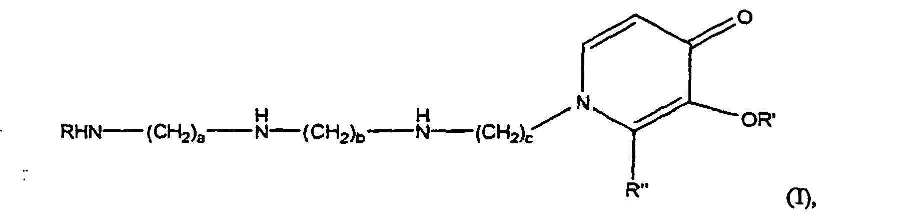 R and R' are preferably each -H and R" is preferably methyl. In one embodiment, when R and R' are each -H and R" is methyl, a, b and c are each independently 3 or 4. In a preferred embodiment, R" is methyl and a, b and c are each 3. In another preferred embodiment, R" is methyl and a is 3, b is 4 and c is 3.

For conjugates of the invention represented by Structural Formula (II): 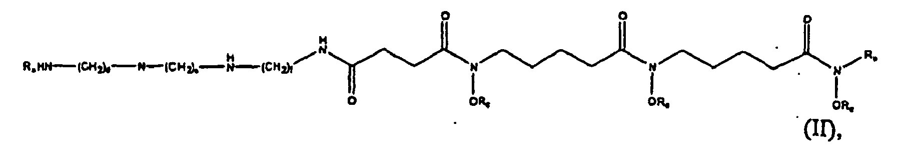 it is preferred that Rₐ and each R_{c} are -H. In one embodiment wherein Rₐ and each R_{c} are -H, d, e and f are each 3 or 4. In one preferred embodiment, d, e and f are each 3. In another preferred embodiment, d is 3, e is 4 and f is 3. In another embodiment, R_{b} is preferably an unsubstituted hydrocarbyl group interrupted by one or more oxygen atoms, such as a 3,6,9-trioxadecyl group. In a particularly preferred embodiment, d, e and f have one of the sets of values described above and R_{b} is a 3,6,9-trioxadecyl group.

For conjugates of the invention represented by Structural Formula (III): 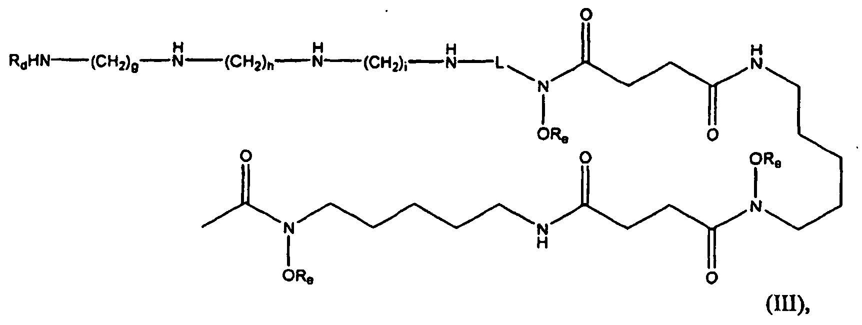 R_{d} and each Rₑ are preferably -H. In one embodiment wherein R_{d} and each Rₑ are -H, g, h and i are each independently 3 or 4. In one preferred embodiment, g, h and i are each 3. In another preferred embodiment, g is 3, h is 4 and i is 3. In another embodiment, L is preferably a substituted or unsubstituted alkylene group, more preferably an unsubstituted alkylene group such as a dimethylene, trimethylene, tetramethylene, pentamethylene, hexamethylene, heptamethylene or octamethylene group. In a particularly preferred embodiment of the invention, g, h and i have one of the sets of values described above and L is a pentamethylene group.

For conjugates of the invention represented by Structural Formula (VII): 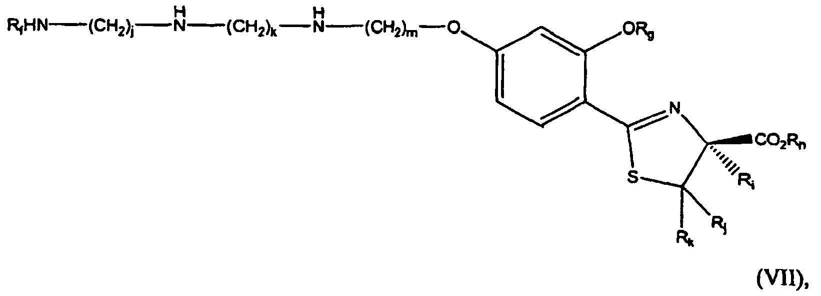 R_{f} is preferably -H. In one embodiment where R_{f} is -H, j and k are independently 3 or 4 and m is an integer from 3 to 6. In one preferred embodiment, j and k are both 3 and m is 4. In another embodiment where R_{f} is -H and j, k and m have the values disclosed above, Rg is -H or an unsubstituted acyl group, preferably -H or an acetyl group, Rₕ is -H or an unsubstituted alkyl group, preferably an ethyl group, Rᵢ is an unsubstituted alkyl group, preferably a methyl group, and Rⱼ and Rₖ are each -H or an unsubstituted alkyl group (e.g., methyl), preferably -H. In a particularly preferred embodiment, j and k are both 3, m is 4, R_{f}, R_{g}, Rⱼ and Rₖ are -H, Rₕ is an ethyl group and Rᵢ is a methyl group.

For treating cancer or a tumor, in one embodiment an iron chelator moiety has five or fewer atoms that coordinate to an iron center. Accordingly, iron chelator moieties for use in treating cancer or a tumor are typically bidentate, tridentate, tetradentate or pentadentate. In another embodiment, an iron chelator moiety has six or more atoms that coordinate to an iron center (e.g., hexadentate and octadentate moieties).

Conjugates of the invention can be co-administered with one or more other active agents in the methods of treatment disclosed herein. One group of active agents that can be co-administered with conjugates of the invention are polyamine synthesis inhibitors. Polyamine synthesis inhibitors include ornithine decarboxylase inhibitors, S-adenosylmethionine inhibitors, spermidine synthase inhibitors and spermine synthase inhibitors. Such polyamine synthesis inhibitors may increase the uptake of the compounds and pharmaceutical compositions of the invention by cells. In general, co-administration of a compound or pharmaceutical composition of the invention is, for example, intended to improve the efficacy of a therapy, increase the rate at which a therapy reduces symptoms of a condition and/or reduce side effects associated with a compound or pharmaceutical composition of the invention.

Ornithine decarboxylase inhibitors include alpha-difluoromethylomithine (DFMO), alpha-monofluoromethylomithine, (2R, 5R)-delta-rnethylacetylenicputrescine, (E)-alpha-monofluoromethyldehydroornithine and its esters (e.g., methyl ester), 3-aminooxy-1-propanamine and 3-(aminooxy)-2-fluoro-1-propanamine.

S-Adenosylmethionine inhibitors include S-(5'-deoxy-5-adenosyl) methylthioethythydroxylamine, 5'-deoxy-5'-[(2-aminooxyethyl)methylamino] adenosine, 5'-deoxy-5'-[(3-hydrazinopropyl)methylamino]adenosine, 5'-{[(Z)-4-amino-2-butenyl]methylamino}-5'-deoxyadenosine, S-(5'-deoxy-5'-adenosyl)-1-amino-4-methylthio-2-cyclopentene, methylglyoxal bis(guanylhydrazone), ethylglyoxal bis(guanylhydrazone), diethylglyoxal bis(guanylhydrazone), [2,2-bipyridine]-6,6-dicarboximidamide and 4-amidinoindan-I-one 2'-amidinohydrazone.

Spermidine synthase inhibitors include S-adenosyl-1,8-diamino-3-thiooctane, cyclohexylamine, *n*-butylamine and trans-4-methylcyclohexylamine.

Spermine synthase inhibitors include S-adenosyl-1,12-diamino-3-thio-9-azadodecane, N-(*n*-butyl)-1,3-diaminopropane and N-(3-aminopropyl)cyclohexylamine.

Conjugates of the invention can also be co-administered with an additional anti-tumor agent. Examples of anti-tumor agents include antimetabolites (e.g., azacitidine, cytarabine, fluorouracil, mercaptopurine, methotrexate, thioguanine, cladribine, fludarabine, allopurinol), bleomycin peptide' antibiotics, podophyllin alkaloids (e.g., etoposide, teniposide), plant alkaloids (e.g., vincristine, vinblastine, paclitaxel, docetaxel), alkylating agents (e.g., busulfan, cyclophosphamide, mechlorethamine, melphalan, thiotepa, chlorambucil, triethylenemelamine), antibiotics (e.g., dactinomycin, danuorubicin, doxorubicin, plicamycin, mitomycin), cis-platin, carboplatin, procarbazine, dacarbazine, altretamine, nitrosoureas (e.g., bis(chloroethyl)nitrosourea (carmustine), 1-(2-chloroethyl)-3-cyclohexyl-1-nitrosourea (lomustine), 1-(2-chloroethyl)-3-(4-methylcyclohexyl)-1-nitrosourea (semustine)), hormonal agents (e.g., androgens such as testosterone and fluoxymesterone, antiandrogens such as flutamide, estrogens such as diethylstilbestrol and ethinyl estradiol, antiestrogens such as tamoxifen, progestins such as hydroxyprogesterone, medroxyprogesterone and megestrol, adrenocorticosteroids such as hydrocortisone and prednisone, gonadotropin-releasing hormone agonists such as goserelin and leuprolide, aromatase inhibitors such as aminoglutethimide and anastrozole, peptide hormone inhibitors such as octreotide) and miscellaneous anti-tumor agents (e.g., amsacrine, asparaginase, hydroxyurea, mitoxantrone, mitotane, retinoic acid derivatives, bone marrow growth factors, amifostine).

Tumors or cancers that can be treated by the invention include, but are not limited to, leukemia, Hodgkin's disease, non-Hodgkin's lymphomas, multiple myeloma, macroglobulinemia, polycythemia vera, lung tumors, head and neck tumors, brain tumors (neuroblastoma), endometrial tumors, ovarian tumors, cervical tumors, breast tumors, choriocarcinoma, testical tumors, prostate tumor, Wilms' tumor, thyroid tumors, adrenal tumors, stomach tumor, pancreal tumors, colonic tumors, carcinoids, insulinoma, bone tumors (osteogenic sarcoma), miscellaneous sarcomas and skin cancer (melanoma).

Subjects suffering from a metal overload condition can be treated with a conjugate that specifically and/or tightly binds to the metal or metals that are present in excess. For example, an iron overload condition is preferably treated with a conjugate having an iron chelator moiety. More preferably, the iron chelator moiety for this indication is hexadentate or greater.

Iron overload conditions or diseases can be characterized by global iron overload or focal iron overload. Global iron overload conditions generally involve an excess of iron in multiple tissues or excess iron located throughout an organism. Global iron overload conditions can result from excess uptake of iron by a subject, excess storage and/or retention of iron, from, for example, dietary iron or blood transfusions. One global iron overload condition is primary hemochromatosis, which is typically a genetic disorder. A second global iron overload condition is secondary hemochromatosis, which is typically the result of receiving multiple blood transfusions. Blood transfusions are often required for subjects suffering from thalassemia or sickle cell anemia. A type of dietary iron overload is referred to as Bantu siderosis, which is associated with high iron content of home-brewed beer.

In focal iron overload conditions, the excess iron is limited to one or a few cell types or tissues or a particular organ. Alternatively, symptoms associated with the excess iron are limited to a discrete organ, such as the heart, lungs, liver, pancreas, kidneys or brain. It is believed that focal iron overload can lead to neurological or neurodegenerative disorders such as Parkinson's disease, Alzheimer's disease, Huntington's disease, neuroferritinopathy, amyotrophic lateral sclerosis and multiple sclerosis.

Wilson's disease is a copper overload condition that can be treated using conjugates of the invention, preferably where the conjugate has a metal chelator moiety that specifically and/or tightly binds copper.

Also included in the present invention are salts and pharmaceutically acceptable salts of the conjugates described herein. Conjugates disclosed herein that possess a sufficiently acidic functional group, a sufficiently basic functional group or both can react with any of a number of organic or inorganic bases, and inorganic and organic acids, to form a salt.

Acidic groups can form salts with one or more of the metals listed above, along with alkali and alkaline earth metals (e.g, sodium, potassium, magnesium, calcium). In addition, acidic groups can form salts with amines. In some embodiments of the invention, it is advantageous to supply a conjugate (e.g., to a subject) as a metal salt. For example, the iron (iron(II) or iron(III)) salt of a conjugate can be administered to a subject. Alternatively, the conjugate is supplied.as a substantially metal-free (e.g. iron-free) salt. Metal-free salts are not typically intended to encompass alkali and alkali earth metal salts. Metal-free salts are advantageously administered to a subject suffering from, for example, a metal overload condition.

Acids commonly employed to form acid addition salts from compounds with basic groups are inorganic acids such as hydrochloric acid, hydrobromic acid, hydroiodic acid, sulfuric acid, phosphoric acid, and the like, and organic acids such as p-toluenesulfonic acid, methanesulfonic acid, oxalic acid, *p*-bromophenyl-sulfonic acid, carbonic acid, succinic acid, citric acid, benzoic acid, acetic acid, and the like. Examples of such salts include the hydroxide, sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, gamma-hydroxybutyrate, glycolate, tartrate, methanesulfonate, propanesulfonate, naphthalene-1-sulfonate, naphthalene-2-sulfonate, mandelate, and the like.

The conjugates disclosed herein can be prepared in the form of their hydrates, such as hemihydrate, monohydrate, dihydrate, trihydrate, tetrahydrate and the like and as solvates.

A "subject" is typically a human, but can also be an animal in need of treatment, e.g., companion animals (e.g., dogs, cats, and the like), farm animals (e.g., cows, pigs, horses, sheep, goats and the like) and laboratory animals (e.g., rats, mice, guinea pigs and the like).

The conjugates or pharmaceutically acceptable salts thereof of the present invention can be administered by an appropriate route. Suitable routes of administration include, but are not limited to, orally, intraperitoneally, subcutaneously, intramuscularly, intradernally, transdernally, rectally, sublingually, intravenously, buccally or via inhalation.

Forms suitable for oral administration include tablets, troches, capsules, elixirs, suspensions, syrups, wafers, chewing gum or the like prepared by art recognized procedures. The amount of active compound in such therapeutically useful compositions or preparations is such that a suitable dosage will be obtained.

The pharmaceutical compositions of the invention preferably contain a pharmaceutically acceptable carrier or diluent suitable for rendering the compound or mixture administrable orally, parenterally, intravenously, intradermally, intramuscularly or subcutaneously, rectally, via inhalation or via buccal administration, or transdermally. The active ingredients may be admixed or compounded with a conventional, pharmaceutically acceptable carrier or diluent. It will be understood by those skilled in the art that any mode of administration, vehicle or carrier conventionally employed and which is inert with respect to the active agent may be utilized for preparing and administering the pharmaceutical compositions of the present invention. Illustrative of such methods, vehicles and carriers are those described, for example, in Remington's Pharmaceutical Sciences, 18th ed. (1990), the disclosure of which is incorporated herein by reference.

The formulations of the present invention for use in a subject comprise the agent, together with one or more acceptable carriers or diluents therefor and optionally other therapeutic ingredients. The carriers or diluents must be "acceptable" in the sense of being compatible with the other ingredients of the formulation and not deleterious to the recipient thereof. The formulations can conveniently be presented in unit dosage form and can be prepared by any of the methods well known in the art of pharmacy. All methods include the step of bringing into association the agent with the carrier or diluent which constitutes one or more accessory ingredients. In general, the formulations are prepared by uniformly and intimately bringing into association the agent with the carriers and then, if necessary, dividing the product into unit dosages thereof.

Formulations suitable for parenteral administration conveniently comprise sterile aqueous preparations of the agents that are preferably isotonic with the blood of the recipient. Suitable carrier solutions include phosphate buffered saline, saline, water, lactated ringers or dextrose (5% in water). Such formulations can be conveniently prepared by admixing the agent with water to produce a solution or suspension, which is filled into a sterile container and sealed against bacterial contamination. Preferably, sterile materials are used under aseptic manufacturing conditions to avoid the need for terminal sterilization.

Such formulations can optionally contain one or more additional ingredients, which can include preservatives such as methyl hydroxybenzoate, chlorocresol, metacresol, phenol and benzalkonium chloride. Such materials are of special value when the formulations are presented in multidose containers.

Buffers can also be included to provide a suitable pH value for the formulation. Suitable buffer materials include sodium phosphate and acetate. Sodium chloride or glycerin can be used to render a formulation isotonic with the blood.

If desired, a formulation can be filled into containers under an inert atmosphere such as nitrogen and can be conveniently presented in unit dose or multidose form, for example, in a sealed ampoule.

Those skilled in the art will be aware that the amounts of the various components of the compositions of the invention to be administered in accordance with the method of the invention to a subject will depend upon those factors noted above.

The compositions of the invention when given orally or via buccal administration can be formulated as syrups, tablets, capsules and lozenges. A syrup formulation will generally consist of a suspension or solution of the compound or salt in a liquid carrier, for example, ethanol, glycerine or water, with a flavoring or coloring agent. Where the composition is in the form of a tablet, one or more pharmaceutical carriers routinely used for preparing solid formulations can be employed. Examples of such carriers include magnesium stearate, starch, lactose and sucrose. Where the composition is in the form of a capsule, the use of routine encapsulation is generally suitable, for example, using the aforementioned carriers in a hard gelatin capsule shell. Where the composition is in the form of a soft gelatin shell capsule, pharmaceutical carriers routinely used for preparing dispersions or suspensions can be considered, for example, aqueous gums, celluloses, silicates or oils, and are incorporated in a soft gelatin capsule shell.

A typical suppository formulation includes the conjugate or a pharmaceutically acceptable salt thereof which is active when administered in this way, with a binding and/or lubricating agent, for example, polymeric glycols, gelatins, cocoa-butter or other low melting vegetable waxes or fats.

Typical transdermal formulations include a conventional aqueous or nonaqueous vehicle, for example, a cream, ointment, lotion or paste or are in the form of a medicated plastic, patch or membrane.

Typical compositions for inhalation are in the form of a solution, suspension or emulsion that can be administered in the form of an aerosol using a conventional propellant such as dichlorodifluoromethane or trichlorofluoromethane.

The therapeutically effective amount of a conjugate or pharmaceutical composition of the invention depends, in each case, upon several factors, e.g., the health, age, gender, size and condition of the subject to be treated, the intended mode of administration, and the capacity of the subject to incorporate the intended dosage form, among others. A therapeutically effective amount of an active agent is an amount sufficient to have the desired effect for the condition being treated. For example, in a method of treating of cancer or a tumor, the desired effect is partial or total inhibition, delay or prevention of the progression of cancer or the tumor including cancer metastasis; inhibition, delay or prevention of the recurrence of cancer or the tumor including cancer metastasis; or the prevention of the onset or development of cancer or a tumor (chemoprevention) in a mammal, for example a human. In a method of treating a subject suffering from a metal overload condition, a therapeutically effective amount of an active agent is, for example, an amount sufficient to reduce the burden of the metal in the subject, reduce the symptoms associated with metal overload or prevent, inhibit or delay the onset and/or severity of symptoms associated with metal overload. In a method of reducing oxidative stress in a subject in need of treatment thereof, a therapeutically effective amount of an active agent is, for example, an amount sufficient to reduce symptoms associated with oxidative stress or prevent, inhibit or delay the onset and/or severity of symptoms associated with oxidative stress.

Typical amounts of a compound of the invention to be administered to a subject are about 500 micrograms to about 5 grams per day (assuming a 70 kg subject). In particular, amounts can range from about I milligram to about 1 gram, for example, about 5 milligrams to about 700 milligrams, such as about 50 milligrams to about 500 milligrams.

An alkyl group is a saturated hydrocarbon in a molecule that is bonded to one other group in the molecule through a single covalent bond from one of its carbon atoms. Alkyl groups can be cyclic or acyclic and branched or unbranched. Typically, an alkyl group has one to about six carbon atoms, or one to about four carbon atoms. Lower alkyl groups have one to four carbon atoms and include methyl, ethyl, *n-*propyl, iso-propyl, *n*-butyl, *sec*-butyl and *tert*-butyl.

An alkylene group is a saturated hydrocarbon in a molecule that is bonded to two other groups in the molecule through single covalent bonds. Alkylene groups can be cyclic or acyclic and branched or unbranched. Typically, an alkylene group has one to about six carbon atoms, or one to about four carbon atoms.

Acyl groups are represented by the formula-C(O)R, where R is a substituted or unsubstituted alkyl group. Acyl groups can be hydrolyzed or cleaved from a compound by enzymes, acids, or bases. One or more of the hydrogen atoms of an acyl group can be substituted, as described below. Typically, an acyl group is removed before a compound of the present invention binds to a metal ion such as iron(III).

Hydrocarbyl groups are monovalent or divalent hydrocarbons. Hydrocarbyl groups can be substituted or unsubstituted, cyclic or acyclic, branched or unbranched and saturated or unsaturated. Hydrocarbyl groups, as indicated, can optionally be interrupted by one or more oxygen atoms in order to form ether linkages. Typically, a hydrocarbyl group contains one to about twelve carbon atoms, one to about six carbon atoms or one to about four carbon atoms.

Suitable substituents for alkyl, alkylene, hydrocarbyl and acyl groups include -OH, -O(R'), -O-CO-(R'), -NO₂, -COOH, =O, -NH₂, -NH(R'), -N(R')₂, -COO(R'), -CONH₂, -CONH(R'), -CON(R')₂ and guanidine. Each R' is independently an alkyl group or an aryl group. These groups can additionally be substituted by an aryl group (e.g., an alkyl group can be substituted with an aromatic group to form an arylalkyl group). A substituted alkyl, alkylene, hydrocarbyl or acyl group can have more than one substituent.

Aryl groups include carbocyclic aromatic groups such as phenyl, p-tolyl, 1-naphthyl, 2-naphthyl, 1-anthracyl and 2-anthracyl. Aryl groups also include heteroaromatic groups such as *N*-imidazolyl, 2-imidazole, 2-thienyl, 3-thienyl, 2-furanyl, 3-furanyl, 2-pyridyl, 3-pyridyl, 4-pyridyl, 2-pyrimidyl, 4-pyrimidyl, 2-pyranyl, 3-pyranyl, 3-pyrazolyl, 4-pyrazolyl, 5-pyrazolyl, 2-pyrazinyl, 2-thiazolyl, 4-thiazolyl, 5-thiazolyl, 2-oxazolyl, 4-oxazolyl and 5-oxazolyl.

Aryl groups also include fused polycyclic aromatic ring systems in which a carbocyclic, alicyclic, or aromatic ring or heteroaryl ring is fused to one or more other heteroaryl or aryl rings. Examples include 2-benzothienyl, 3-benzothienyl, 2-benzofuranyl, 3-benzofuranyl, 2-indolyl, 3-indolyl, 2-quinolinyl, 3-quinolinyl, 2-benzothiazole, 2-benzooxazole, 2-benzimidazole, 2-quinolinyl, 3-quinolinyl, 1-isoquinolinyl, 3-quinolinyl, 1-isoindolyl and 3-isoindolyl.

Cleavable functional groups can be cleaved either chemically or enzymatically. Preferably, a cleavable functional group is hydrolyzable. Examples of cleavable functional groups include ester, amide, carbamate, carbonate, dithiocarbamate, phosphate ester and phosphite ester groups.

Linking groups, as used herein, connect the metal chelator moiety of a conjugate to the polyamine moiety of the conjugate. A linking group typically has suitable functional groups at each end in order to enable the two moieties to be attached. Linking groups are chosen such that they do not deleteriously affect the metal chelating activity of the metal chelator moiety or the pKₐ of the nitrogen atoms of the polyamine moiety. A linking group can optionally contain one or more cleavable functional groups, so as to permit release of one or both of the metal chelator moiety and the polyamine moiety from the conjugate. Preferably, release of either or both moieties occurs within a cell. Suitable linking groups include hydrocarbyl groups, as described above. Preferably, a linking group is an alkylene group such as a pentamethylene group.

No linking group is present in a conjugate if a metal chelator moiety is directly attached to a polyamine moiety by a covalent bond. An example of such a conjugate is shown in Example 1. In one type of conjugate where the polyamine and metal chelator moieties are directly connected, the polyamine moiety cannot be cleaved from the metal chelator moiety as a single unit (although aminopropyl fragments can be individually cleaved).

### EXEMPLIFICATION

The present invention will now be illustrated by the following Examples, which are not intended to be limiting in any way.

### Example 1

### Synthesis of 1-(12-Amino-4,9-diazadodecyl)-2-methyl-3-hydroxy-4(1H)-pyridinone Tetrahydrochloride (Compound 3)

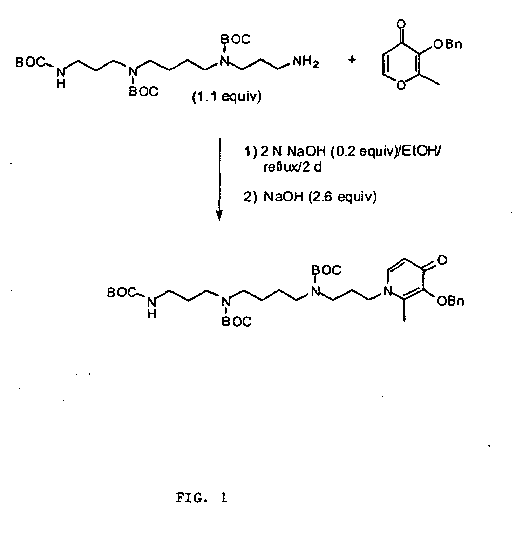

The synthesis is represented schematically in FIG. 1.

Reagents were purchased from Aldrich Chemical Co. (Milwaukee, WI), and Fisher Optima-grade solvents were routinely used. Silica gel 32-63 from Selecto Scientific, Inc. (Suwanee, GA) was used for flash column chromatography, and Sephadex LH-20 was obtained from Amersham Biosciences (Piscataway, NJ). Melting points are uncorrected. NMR spectra were obtained at 300 MHz (¹H) or 75 MHz (¹³C) on a Varian Unity 300 in D₂O, with chemical shifts (δ) given in parts per million referenced to sodium 3-(trimethylsilyl)propionate-2,2,3,3-*d*₄ (0.0) or 1,4-dioxane (67.19), respectively. Coupling constants (*J*) are in hertz. Elemental analyses were performed by Atlantic Microlabs (Norcross, GA).

### 1-(12-Amino-4,9-diazadodecyl)-2-methyl-(phenymethoxy)-4(1H)-pyridinone Tetrahydrochloride (Compound 2)

Sodium hydroxide (2 N, 190 mL, 0.38 mol) was added in portions to a solution of 3-benzyloxy-2-methyl-4-pyrone (Compound 1) (11.49 g, 53.15 mmol) and spermine·4HCl (20.20 g, 58.01 mmol) in 39% aqueous EtOH (570 mL) with ice bath cooling. The reaction mixture was stirred at room temperature for 1 day, and its volume was reduced by rotary evaporation. Water (200 mL) was added, followed by several CHCl₃ extractions. The organic portion was washed with saturated Nad, dried with sodium sulfate, and concentrated under reduced pressure. Flash chromatography (30% concentrated NH₄OH/CH₃OH) and acidification with concentrated HCI in EtOH gave Compound 2 (3.35 g, 12%) as a white solid: mp 188-190 °C; ¹H NMR δ 1.7-1.8 (m, 4 H), 2.0-2.2 (m, 4 H), 2.28 (s, 3 H), 3.0-3.2 (m, 10 H), 4.23 (t, 2 H, *J =* 7.7), 5.12 (s, 2 H), 6.91 (d, I H, *J =* 7.2), 7.4-7.5 (m, 5 H), 7.98 (d, 1 H, J= 7.5); ¹³C NMR δ 13.5,23.4,24.4,26.8,37.2,44.8,45.2,47.6, 47.7, 53.7, 76.0, 114.3, 129.5, 129.9, 130.3, 135.8, 142.5, 143.7, 150.6, 165.9. Anal. (C₂₃H₄₀Cl₄N₄O₂·2H₂O) C, H, N.

### 1-(12-Amino-4,9-diazadodecyl)-2-methyl-3-hydroxy-4(1H)-pyridinone Tetrahydrochloride (Compound 3)

Distilled solvents and glassware that had been presoaked in 3 N HCI for 15 min were employed. Pd-C (10%, 400 mg) was added to Compound 2 (1.43 g, 2.62 mmol) in 38% aqueous CH₃OH (130 mL). The reaction mixture was stirred under H₂ at 1 atm for 5 h and was filtered through Celite, which was washed with water (10 mL) and EtOH (30 mL). After removal of the solvents in vacuo, chromatography on Sephadex LH-20 (EtOH) furnished Compound 3 (0.911 g, 76%) as a white solid: mp 216-218 °C; ¹H NMR δ1.7-1.8 (m, 4 H), 2.02-2.15 (m, 2 H), 2.18-2.31 (m, 2 H), 2.57 (s, 3 H), 3.0-3.2 (m, 10 H), 4.39 (t, 2 H,*J*= 7.6), 6.99 (d, 1 H, J = 7.0), 7.97 (d, 1 H, J = 7.0); ¹³C NMR δ 12.8, 23.4, 24.4, 26.9, 3 7.2, 44.9, 45.2, 47.6, 53.8, 112.0, 139.0, 142.5, 143.6,160.1. Anal. (C₁₆H₃₄Cl₄N₄O₂·H₂O) C, H, N.

In a synthesis of Compound 3 designed to direct ring formation at a single primary amine, *N*¹,*N*⁴,*N*⁹-tris(*tert*-butoxycarbonyl)spermine was heated with Compound I and NaOH in aqueous EtOH, generating the tris(BOC) derivative of Compound 2 in 9% yield.

### Example 2

### Stoichiometry of Metal-Ligand Complexes

1,2-Dimethyl-3-hydroxypyridin-4-one (L1) was a generous gift from Dr. H. H. Peter (Ciba-Geigy, Basel, Switzerland).

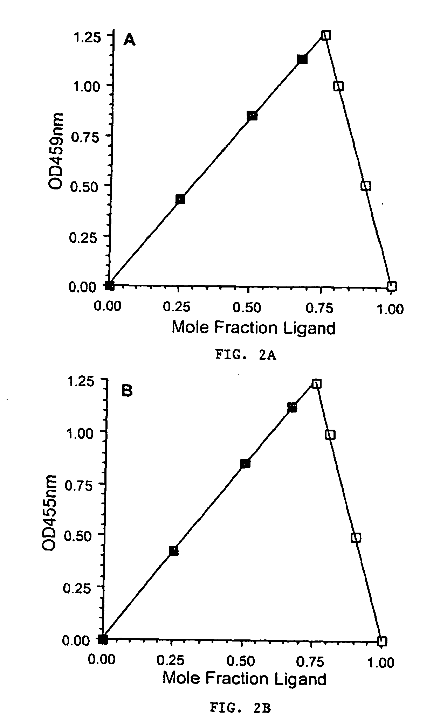

The stoichiometry of a metal-ligand complex was determined spectrophotometrically for Compound 3 and L1 at the λₘₐₓ (FIGS. 2A and 2B, 459 and 455 nm, respectively) of the visible absorption band of the ferric complexes. Briefly, a 0.5 mM Fe(III) nitrilotriacetate (NTA) solution was made immediately before use by dilution of a 50 mM Fe(III)-NTA stock solution with TRIS buffer. Solutions of the ferric complex containing different ligand/Fe(III) ratios were then prepared by mixing appropriate volumes of 0.5 mM ligand in 100 mM TRIS Cl, pH 7.4, and 0.5 mM Fe(III)-NTA such that the combined concentration of ligand and Fe(III) was a constant at 1.00 mM. The Job's plot for each set of mixtures was then derived.

The plots for Compound 3 and L1 were essentially identical, demonstrating that both compounds formed a 3:1 ligand/metal complex. These results clearly indicated that the spermine fragment of the L1-polyamine conjugate Compound 3 does not alter the stoichiometry of the ferric complex.

### Example 3

### Effect of a Conjugate on Cell Proliferation

*N*¹-Acetylspermine (AcSPM) as its trihydrochloride salt (A-2679) was purchased from Sigma (St. Louis, MO).

Murine L1210 leukemia cells were maintained in logarithmic growth as a suspension culture in RPMI-1640 medium (Gibco, Grand Island, NY) containing 10% fetal bovine serum (Gibco), 2% HEPES-MOPS buffer, 1 mM L-glutamine (Gibco), and 1 mM aminoguanidine at 37 °C in a water-jacketed 5% CO₂ incubator.

Cells were grown in 25 cm² tissue culture flasks in a total volume of 10 mL. Cultures were treated during logarithmic growth (0.5-1.0 × 10⁵ cells/mL) with the compounds of interest, reseeded, and incubated as described in Bergeron, R. J., Müller, R., Bussenius, J., McManis, J. S., Merriman, R. L., Smith, R. E., Yao, H., Weimar, W. R., "Synthesis and Evaluation of Hydroxylated Polyamine Analogues as Antiproliferatives," J. Med. Chem 43: 224-235 (2000). Cell counting and calculation of percent of control growth were also carried out as described in Bergeron, *et al.* The IC₅₀ is defined as the concentration of compound necessary to reduce cell growth to 50% of control growth after defined intervals of exposure.

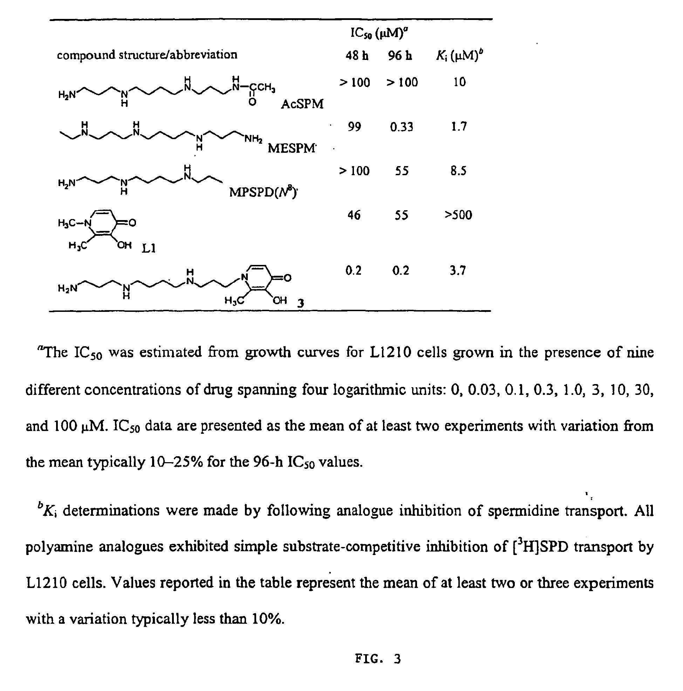

Several different polyamines were tested for their effect on cell proliferation (FIG. 3). *N*¹-acetylspermine (AcSPM), a trication; *N*¹-ethylspermine (MESPM), a tetracation; and *N*⁸-propylspermidine [MPSPD(*N*⁸)], another trication. In each of these polyamines, one end of the molecule remains a primary amine affixed to an aminopropyl group, as in the conjugate 3. In each case, the 48- and 96-h IC₅₀ and *K*ᵢ values are presented. The numbers for MESPM and MPSPD(*N*⁸) are historical; AcSPM, L1, and compound 3 were tested in the current series of experiments. After 48 h of treatment, none of the three model polyamine analogues have significant IC₅₀ values, 99 µM for MESPM and greater than 100 µM for MPSPD(*N*⁸) and AcSPM. The situation is remarkably different at 96 h for MESPM (0.33 µM); neither MPSPD(*N*⁸) nor AcSPM is particularly active (55 and > 100 µM, respectively). The parent iron chelator, L1, is not especially efficacious at either 48 (46 µM) or 96 (55 µM) h. However, conjugate 3 is quite effective. The IC₅₀ value is 0.2 µM at both time points, at least 230 times more active than the parent ligand. At 48 h, compound 3 is nearly 500 times more active than any of the parent polyamine analogues, although by 96 h its IC₅₀ value is comparable to that of MESPM. The most profound difference between the conjugate and the polyamine analogues is that whereas the IC₅₀ values of the alkyl polyamine analogues diminish as the length of treatment decreases, the IC₅₀ value for the conjugate is low at 48 h and remains the same at 98 h. This behavior is similar to that of the chelator moiety L1, which remains static in its behavior, even though the corresponding IC₅₀ values are much higher than those for compound 3.

### Example 4

### Competition for the Polyamine Transporter

The compounds of interest were studied for their ability to compete with [³H]SPD for uptake into L1210 leukemia cell suspensions in vitro as described in Bergeron, R. J., Hawthorne, T. R., Vinson, J. R. T., Beck, D. E., Jr., Ingeno, M. J., "Role of the Methylene Backbone in the Antiproliferative Activity of Polyamine Analogues on L1210 Cells," Cancer Res. 49: 2959-2964 (1989); Bergeron, R. J., McManis, J. S., Liu, C. Z., Feng, Y., Weimar, W. R., Luchetta, G. R., Wu, Q., Ortiz-Ocasio, J., Vinson, J. R. T., Kramer, D., Porter, C., "Antiproliferative Properties of Polyamine Analogues: A Structure-Activity Study," J. Med. Chem. 37: 3464-3476 (1994); and Bergeron, R. J., Weimar, W. R., Wu, Q., Feng, Y., McManis, J. S., "Polyamine Analogue Regulation of NMDA MK-801 Binding: A Structure-Activity Study," J. Med. Chem. 39: 5257-5266 (1996). Briefly, cell suspensions were incubated in 1 mL of culture medium containing radiolabeled SPD alone or radiolabeled SPD in the presence of graduated concentrations of a chelator or derivative. At the end of the incubation period, the tubes were centrifuged; the pellet was washed, digested, and neutralized prior to scintillation counting. Lineweaver-Burk plots indicated a simple competitive inhibition with respect to SPD.

Each of the polyamine analogues competed effectively for the polyamine transport apparatus. The *K*ᵢ values were 10, 1.7, and 8.5 µM for AcSPM, MESPM, and MPSPD(*N*⁸), respectively (FIG. 3). Although the parent ligand L1 was not a transport competitor, its spermine conjugate 3 was a very effective transport competitor (*K*ᵢ, 3.7 µM), suggesting that the polyamine does serve as a vector for the ligand (FIG. 3).

### Example 5

### Effect of Conjugates on Polyamine Pools and Accumulation of Analogues

During logarithmic growth, L1210 leukemia cells were treated with the compounds of interest at the 48 hour IC₅₀ concentration. At the end of the treatment period, cell suspensions were sampled, washed three times in ice-cold, incomplete medium, and pelleted for extraction using 0.6 N perchloric acid, then freeze-fractured in liquid nitrogen/hot water three times. Each supernatant was frozen at -20 °C until analysis of polyamine content by HPLC. L1 was measured by an HPLC method with UV detection described in the literature with the following modifications: mobile phase A, 20% buffer/80% CH₃OH; mobile phase B, 98% buffer/2% CH₃CN. The buffer consisted of potassium phosphate (10 mM) and EDTA (2 mM), pH 2.9. Under these conditions, the polyamines eluted on the chromatogram as follows (min): putrescine, 10.8; AcSPM, 27.3; spermidine, 33.1; compound 3, 47.1; spermine, 48.0.

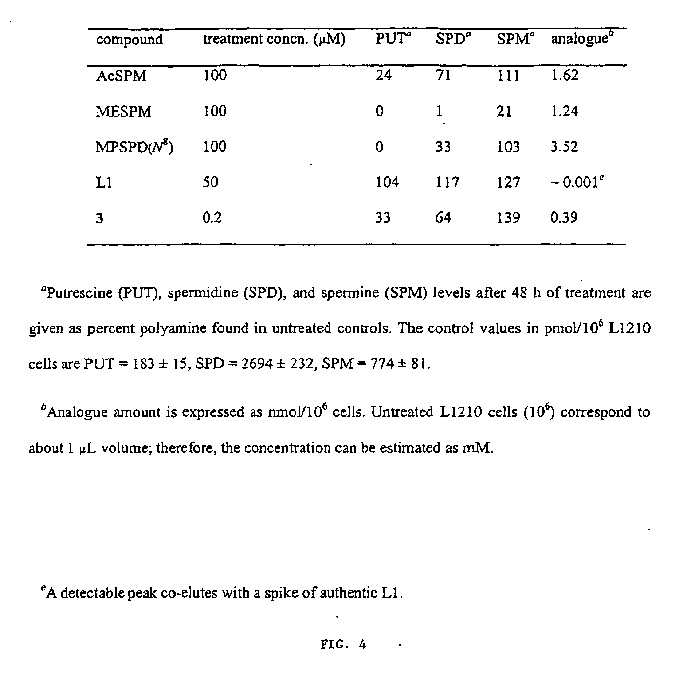

The effects on the native polyamines are reported as the percent of the polyamine found in untreated control cells, and the analogue accumulation is reported as mM (FIG. 4). Of all four polyamine analogues, including the conjugate 3, MESPM was the most effective at reducing polyamine pools. At a treatment concentration of 100 µM, putrescine, spermidine, and spermine were diminished to 0, 1, and 2 1 % of control, respectively. At the same treatment concentration, MPSPD(*N*⁸) lowered putrescine and spermidine, although spermine was unaffected (103%). However, at a treatment concentration of 100 µM, AcSPM had a substantial effect on putrescine, generated a slight decrease in spermidine, and increased spermine levels. Of these three polyamines, MPSPD(*N*⁸) achieved the highest intracellular concentration, 3.52 mM; AcSPM and MESPM reached concentrations less than half of this, 1.62 and 1.24 mM, respectively. Perhaps not surprisingly, treatment of cells with L1 (50 µM) had little impact on native polyamines (FIG. 4); putrescine and spermidine remained close to control levels, and spermine rose to 127% of control values. The behavior of conjugate 3 when given at its IC₅₀ concentration (0.2 µM) was vastly different from that of L1 and very similar to that of AcSPM. Putrescine was diminished to 33% of control, spermidine was reduced to 64% of control, and spermine increased to 139% of control values. The conjugate 3 was transported quite efficiently into the cells; the intracellular concentration of 0.39 mM is more than 1900-fold higher than the extracellular treatment concentration and is also several hundred times higher than the intracellular concentration attained by the parent ligand L1.

### Example 6

### Effects on Polyamine Metabolic Enzymes

Ornithine decarboxylase (ODC) and Adenosylmethionine decarboxylase (AdoMet DC) activities were determined according to the procedures of Seely, J. E., Pegg, A. E., "Omithine Decarboxylase (Mouse Kidney)," Methods Enzymol. 94: 158-161 (1983) and Pegg, A. E., Pösö, H., "S-Adenosylmethionine Decarboxylase (Rat Liver)," Methods Enzymol. 94: 234-239 (1983), respectively, on the basis of quantitation of ¹⁴CO₂ released from [¹⁴C]carboxyl-labeled L-ornithine or S-adenosyl-L-methionine. Included in each assay were untreated L1210 cells as negative controls as well as cells treated with DEHSPM, a drug having a known reproducible effect on each enzyme, as positive controls.

Spermidine/spermine *N*¹-acetyl transferase (SSAT) activity was based on quantitation of [¹⁴C]-*N*¹-acetylspermidine formed by acetylation of SPD with [¹⁴C]-acetyl coenzyme A according to the method of Libby, P. R., Bergeron, R. J., Porter, C. W., "Structure-Function Correlations of Polyamine Analog-Induced Increases in Spermidine/Spermine Acetyltransferase Activity," Biochem. Pharmacol. 38: 1435-1442 (1989). Cells treated with DENSPM were positive controls.

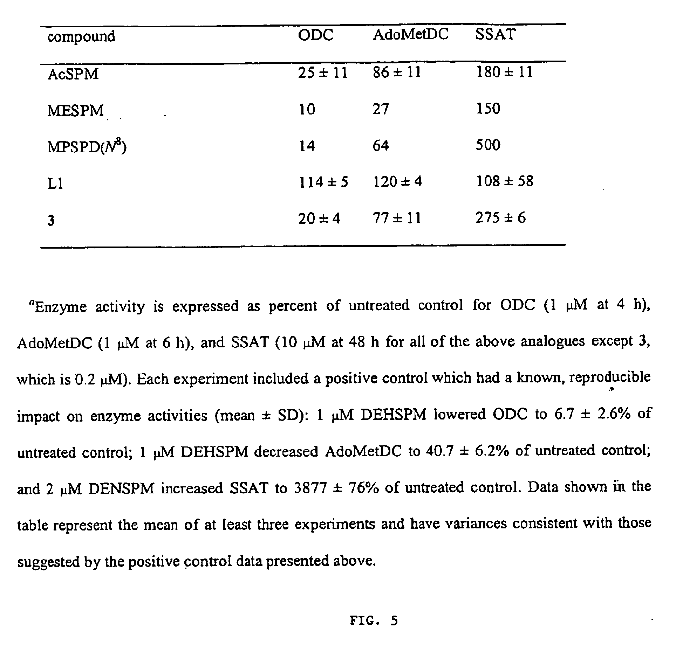

Both MESPM and MDSPD(*N*⁸) exerted rather significant effects on all three polyamine enzymes (FIG. 5). MESPM diminished ODC and AdoMetDC to 10% and 27% of control, respectively, and slightly increased SSAT activity to 150% of control. Although its impact on ODC was similar to that of MESPM, MPSPD(*N*⁸) decreased AdoMetDC to a lesser extent (64% of control) and substantially augmented SSAT activity, to 500% of control. In the case of AcSPM, its impact on ODC was comparable to that of the other two polyamines, 25 ± 11% of control, but its effect on AdoMetDC was not as great (86 ± 11%); this polyamine boosted SSAT activity to 180 ± 11% of control. It was not unexpected that the parent chelator L1 did not substantially influence the activity of any of the polyamine enzymes (ODC, 114 ± 5%; AdoMetDC, 120 ± 4%; SSAT, 108 ± 58%). The conjugate 3 behaved much like MPSPD(*N*⁸), reducing ODC and AdoMetDC to 20 ± 4% and 77 ± 11% of control, respectively, and increasing SSAT activity to 275 ± 6% of control. These results further support the idea of incorporation of the conjugate.

### Example 7

### Preparation of a Spermine Conjugate of a Triether Amideless Analogue of Desferrioxamine

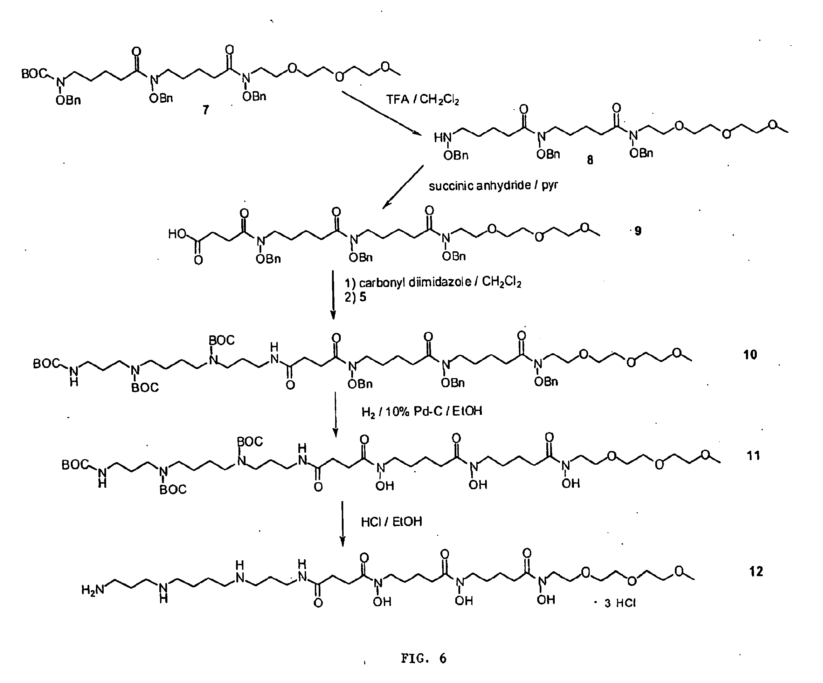

This reaction sequence is shown schematically in FIG. 6. Trihydroxamic acid reagent 7 was converted to *O*-benzyl hydroxylamine 8 using trifluoroacetic acid (TFA) in CH₂Cl₂. A four-carbon spacer was appended to 8 with succinic anhydride in pyridine, forming carboxylic acid 9. Acid 9 was activated with carbonyl diimidazole and treated with *N*¹,*N*⁴,*N*⁹-tris-(*tert*-butoxycarbonyl)spermine (5), yielding fully protected chelator 10. Hydrogenolysis of the benzyl groups of 10 and purification using Sephadex LH-20 gave trihydroxamate 11, in which the amines remain protected. Treatment of 11 with ethanolic HCI produced triamino chelator 12. Stirring 11 with TFA effected clean deprotection of the amino groups, and ion exchange provided trihydrochloride salt 12.

### Example 8

### Effect of a Spermine Conjugate of a Triether Amideless Analogue of Desferrioxamine on Cell Proliferation

The effect of analogue 12 (Example 7) on the growth of L1210 murine leukemia cells (IC₅₀) and its ability to compete with radiolabeled spermidine for the polyamine transport apparatus (*K*ᵢ) was tested using the method described in Example 4. The 96-hour IC₅₀ value was 1.5 µM, which is comparable to many tetraamine analogues. The *K*ᵢ value was 7 µM, which indicated good competition for the polyamine transport apparatus.

### Example 9

### Preparation of a Norspermidine-Desferrioxamine Conjugate

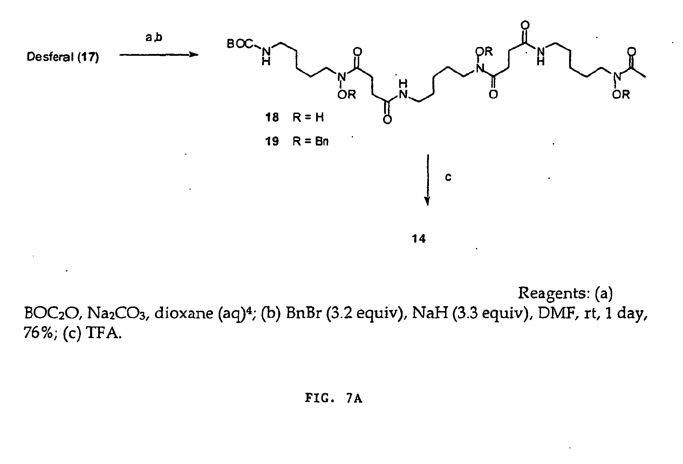

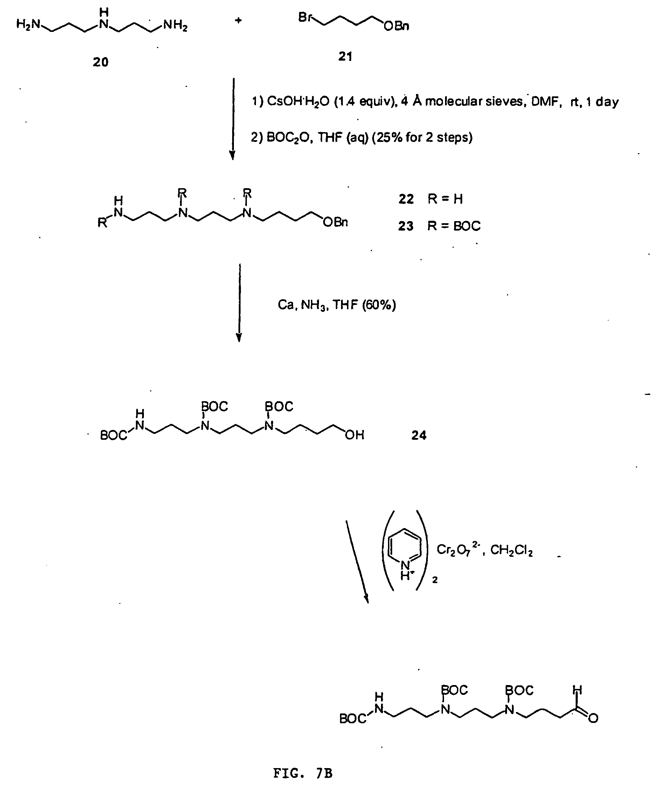

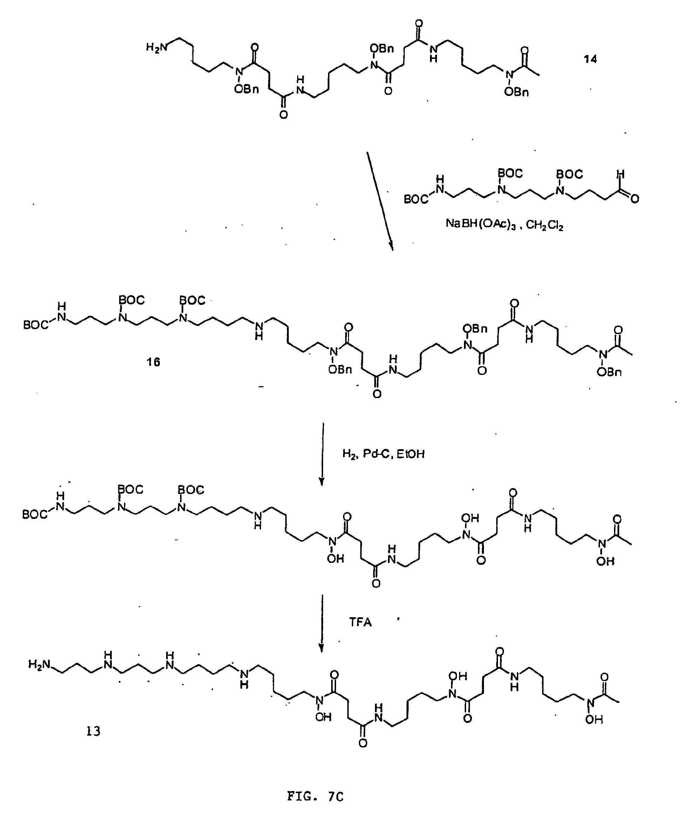

The reaction sequence is shown schematically in FIGS. 7A-7C. Desferal (17) was converted into *N*-BOC derivative 18 by reaction with di(*t*-butyl) dicarbonate, sodium carbonate and aqueous dioxane. The hydroxamates of 18 were benzylated at room temperature over a day with 3.2 equivalents of benzyl bromide and 3.3 equivalents of sodium hydride in dimethylformamide (DMF), producing 3 g of tetraprotected DFO reagent 19. Partial deprotection of 19 with trifluoroacetic acid gave *O*,*O*,*'O"*-tribenzyldesferrioxamine (14).

Norspermidine (20) was reacted with benzyl 4-bromobutyl ether (21, 1.2 equiv) over a day at room temperature in the presence of 1.4 equivalents of cesium hydroxide monohydrate and 4 A molecular sieves in DMF to give 22. The amino groups were protected using di(*t*-butyl) dicarbonate in aqueous tetrahydrofuran (THF) to give 23. Calcium in ammonia and THF efficiently generated alcohol 24. The alcohol was converted into an aldehyde using pyridinium dichromate in CH₂Cl₂.

The aldehyde and *O,O',O"*-tribenzyldesferrioxamine (14) were connected using presence of NaBH(CH₃CO₂)₃ in CH₂Cl₂ to produce 16. 16 was partially deprotected with H₂ and Pd-C in ethanol. Deprotection was completed with trifluoroacetic acid to yield norspermidine-desferrioxamine conjugate 13.

The conjugate 13 was tested in an IC₅₀ assay as described in Example 3 and was determined to be 80 micromolar. The Kᵢ for conjugate 13 was measured as described in Example 4 and was found to be 5 micromolar.

### Example 10

### Preparation of a Norspermidine-4'-Hydroxydesazadesferrithiocin Conjugate (Norspermidine-(S)-4'-(OH)-DADFT Conjugates)

Reagents were purchased from Aldrich Chemical Co. (Milwaukee, WI), and Fisher Optima-grade solvents were routinely used. Silica gel 32-63 from Selecto Scientific, Inc. (Suwanee, GA) was used for flash column chromatography, and Sephadex LH-20 was obtained from Amersham Biosciences (Piscataway, NJ). Melting points are uncorrected. NMR spectra were obtained at 300 MHz (¹H) or 75 MHz (¹³C) on a Varian Unity 300 in D₂O, with chemical shifts (δ) given in parts per million referenced to sodium 3-(trimethylsilyl)propionate-2,2,3,3-*d*₄ (0.0) or 1,4-dioxane (67.19), respectively. Coupling constants (*J*) are in hertz. Elemental analyses were performed by Atlantic Microlabs (Norcross, GA).

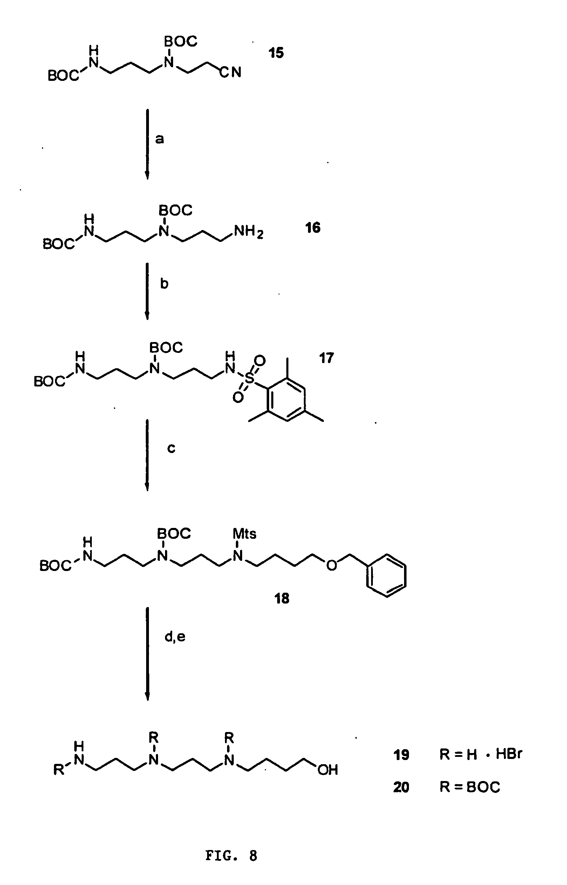

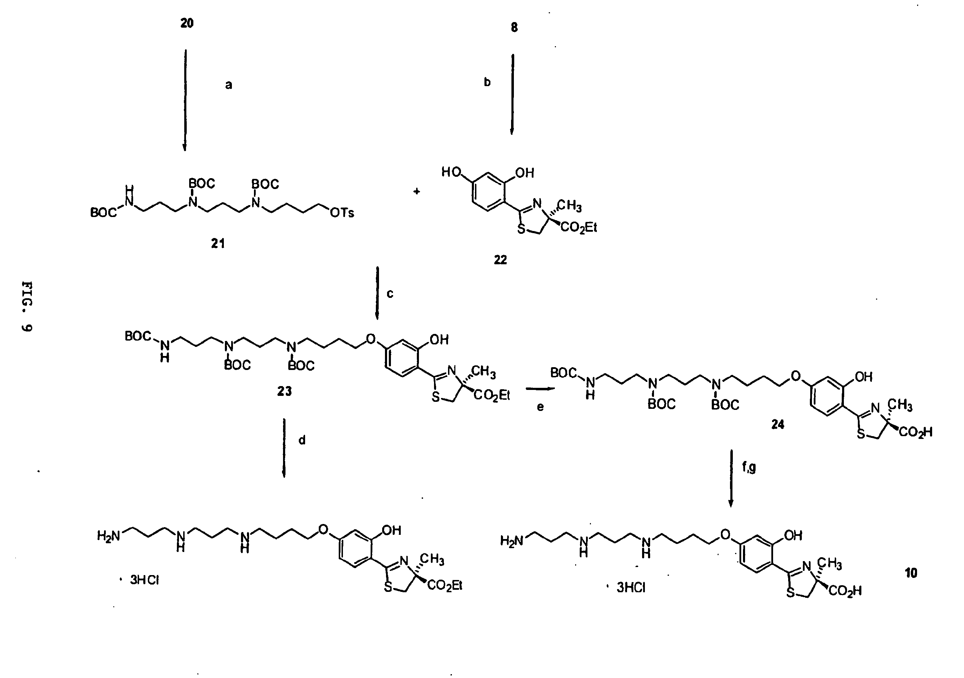

The reaction scheme to prepare the Norspermidine-(*S*)4'-(HO)-DADFT conjugates (Compounds 9 and 10) shown in FIG. 9 is depicted in FIGS. 8 and 9. The first steps of the synthesis involved preparation of a protected triamine with a 4-hydroxybutyl connector at *N*¹ (20), as shown in FIG. 8. Bis(BOC)aminonitrile 15 was hydrogenated over Raney nickel under highly alkaline conditions in ethanolic NaOH to *N*¹*,N*⁴-bis(*tert*-butoxycarbonyl)norspermidine (16). The ω-hydroxy tether was affixed to the amine end of 16 in two steps. Mesitylenesulfonyl chloride was reacted with 16 in aqueous NaOH and CH₂Cl₂ (85% yield for two steps), and the resulting sulfonamide 17 was alkylated with 1.1 equivalents of benzyl 4-bromobutyl ether in the presence ofNaH and DMF, giving benzyl ether 18 in 78% yield. The three nitrogens and the oxygen of 18 were unmasked by employing 30% HBr in acetic acid and phenol in CH₂Cl₂, and the amino groups of trihydrobromide salt 19 were trapped as their *tert*-butyl carbamates using di-(*t*-butyl)-dicarbonate in THF and triethylamine, furnishing *N*¹-(4-hydroxybutyl)-*N*¹,*N*⁴,*N*⁷-tris(*tert-*butoxycarbonyl)norspermidine (20) in 59% yield from 18.

The alcohol group of NSPD reagent 20 was activated in 80% yield as tosylate 21 with tosyl chloride in triethylamine and CH₂Cl₂ (FIG. 9). (*S*)-4'-(HO)-DADFT (8) was converted to ethyl (*S*)-2-(2,4-dihydroxyphenyl)-4,5-dihydro-4-methyl-4-thiazolecarboxylate (22) in 92% yield using iodoethane and *N,N-*diisopropylethylamine (DIEA) in DMF. Deprotonation of 22 using sodium ethoxide and heating at 60°C with tosylate 21 in ethanol effected monoalkylation of the resorcinol system in 67% yield. Structural assignment of 23 was based on selective 4-*O*-alkylation of 2,4-dihydroxycarboxyl systems and on NOESY of final product 10. Irradiation at δ 4.18, corresponding to the α-methylene of the ether function, significantly enhanced the aromatic signals at δ 6.59 and 6.69, as well as a p-methylene of the linear chain. Similarly, NOE correlations from the methyl group of 2-hydroxy-4-methoxybenzaldehyde to the adjacent protons on the aromatic ring (H-3 and H-5) were observed. However, only one ring proton (H-3) was so affected in the analogous experiment with 4-hydroxy-2-methoxybenzaldehyde. Thus the 4'-position of the *O*-alkyl group of 23 in FIG. 9 was confirmed. Treatment of 23 with ethanolic HCl, generated *in situ* from acetyl chloride, removed the BOC groups, affording the ethyl ester of the NSPD-(*S*)-4'-(HO)-DADFT conjugate (9 of FIG. 9) (75%). Saponification of the ester in 23 with I N NaOH in aqueous methanol resulted in tris(BOC) acid 24 in 70% yield. The carbamates of 24 were cleaved with trifluoroacetic acid (TFA) in CH₂Cl₂, followed by ion exchange chromatography, providing (*S*)-4,5-dihydro-2-[2-hydroxy-4-(12-amino-5,9-diazadodecyloxy)phenyl]-4-methyl-4-thiazolecarboxylic acid trihydrochloride (10 of FIG. 9) in 68% yield.

### Example 11

### Iron-Binding Stoichiometry of a Norspermidine-4'-Hydroxydesazadesferrithiocin Conjugate (Compound 10 of FIG. 9)

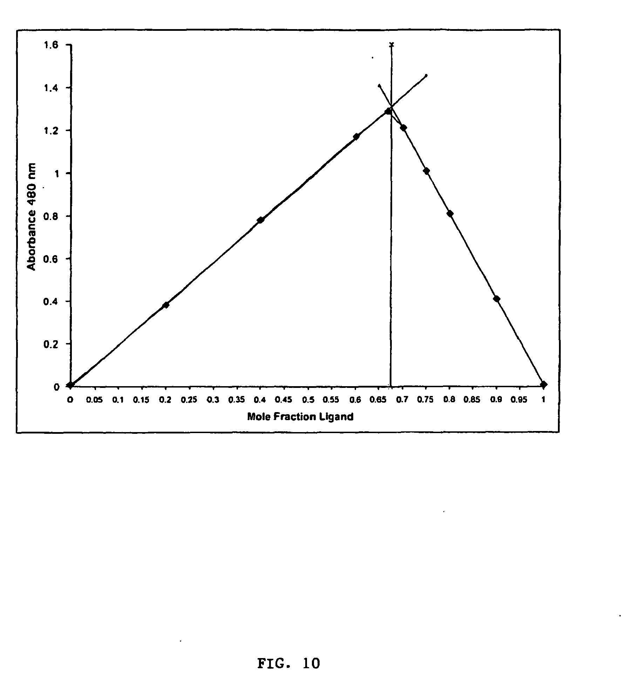

This assay was carried out using the procedure described in Example 2. The stoichiometry of the ferric complexes of (*S*)-4,5-dihydro-2-[2-hydroxy-4-(12-amino-5,9-diazadodecyloxy)phenyl]-4-methyl-4-thiazolecarboxylic acid trihydrochloride was determined spectrophotometrically at its λₘₐₓ, 480 nm. Briefly, solutions containing different ligand/Fe(III) ratios were prepared so that [ligand] + [Fe(III)] = 1.0 mM. The data points were fitted to the mole fractions (1) from 0 to 0.60 and (2) from 0.70 to 1.00; *r*² = 0.9997 (4 data points) and .9999 (5 data points), respectively. The theoretical mole fraction maximum for a 2:1 ligand/Fe complex is 0.667; a linear intercept maximum of 0.675 was found. The Job's plot of the conjugate (FIG. 10) is consistent with a 2:1 ligand/metal complex for this conjugate, the same as for the non-conjugated counterpart.

### Example 12

### Effect of a Norspermidine-4'-Hydroxydesazadesferrithiocin Conjugate on Cell Proliferation

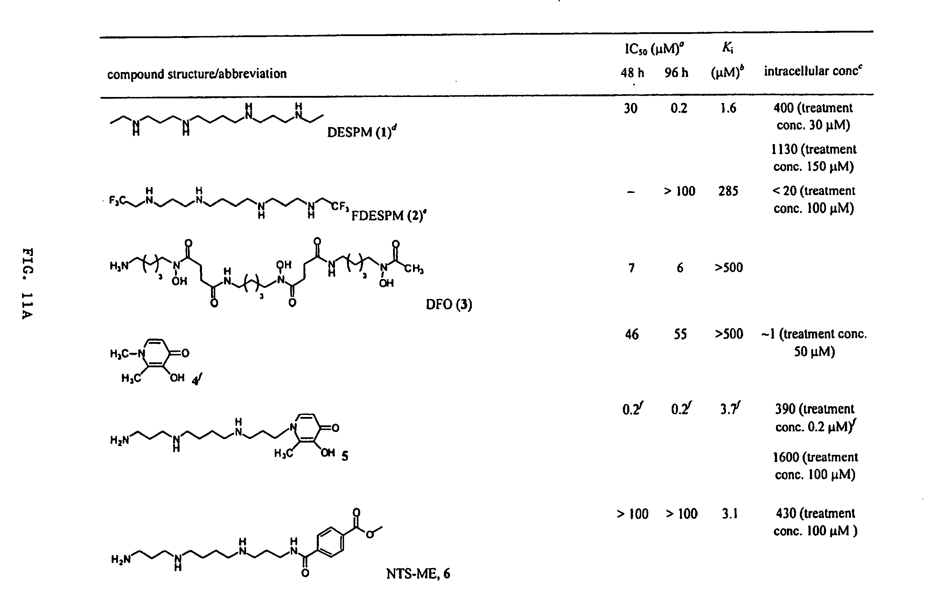

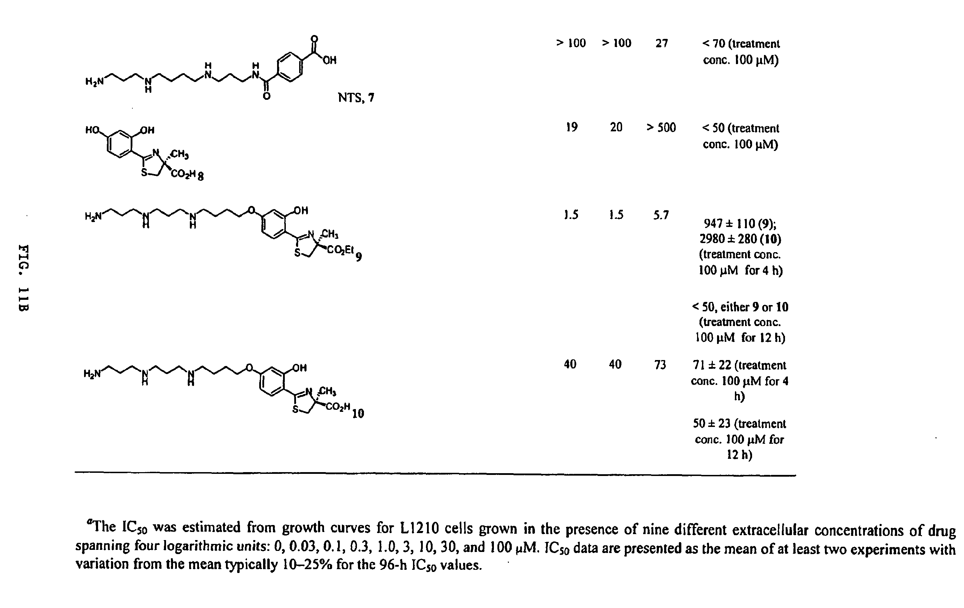

The IC₅₀ values were determined at both 48 and 96 h in L1210 cells (FIGS. 11A and 11B). The values for DESPM (1), FDESPM (2), L1 (4), and the SPM-L1 conjugate (5) are included in FIGS. 11A and 11B for comparative purposes. Although FDESPM (2) is not active in the IC₅₀ assay (> 100 µM), the behavior of DESPM (1) in the IC₅₀ assay is typical of many polyamine analogues, that is, the IC₅₀ decreases as the length of treatment increases, in this case, from 30 µM at 48 h to 0.2 µM at 96 h.

Murine L1210 leukemia cells were maintained in logarithmic growth as a suspension culture in RPMI-1640 medium (Gibco, Grand Island, NY) containing 10% fetal bovine serum (Gibco), 2% HEPES-MOPS buffer, 1 mM L-glutamine (Gibco), and 1 mM aminoguanidine at 37 °C in a water-jacketed 5% CO₂ incubator.

Iron chelators typically maintain a constant IC₅₀ value as treatment time progresses. For example, at the same time points, DFO (3) has IC₅₀ values of 7 µM (48 h) and 6 µM (96 h); the L1 (4) IC₅₀ values are somewhat higher, 46 and 55 µM at 48 and 96 h, respectively. Appending a polyamine vector to L1 (5) decreased the IC₅₀ considerably, to 0.2 µM at both 48 and 96 h.

Consistent with these findings, the IC₅₀ values for 4'-hydroxydesazadesferrithiocin ((*S*)-4'-(HO)-DADFT (8)) at 48 and 96 h were within error of each other, 19 and 20 µM, respectively. Although affixing norspermidine (NSPD) to the 4'-hydroxyl of (*S*)-4'-(HO)-DADFT (8) via a butyl linkage to afford NSPD-(*S*)-4'-(HO)-DADFT (10) increased the 48- and 96-h IC₅₀ values to 40 µM, conversion of this conjugate to the corresponding ethyl ester (9) decreased the IC₅₀ to 1.5 µM at both time points. Neither of the terephthalate analogues, *N*¹-terephthaloylspermidine methyl ester (NTS-ME, 6), or *N*¹-terephthaloylspermidine (NTS, 7) had any impact on cell growth; the 48- and 96-h IC₅₀ values for both compounds were > 100 µM.

### Example 13

### Role of Charge in Competition for the Polyamine Transporter

The molecules of interest were studied for their ability to compete with [³H]SPD for uptake into L1210 leukemia cell suspensions *in vitro.* Briefly, cell suspensions were incubated in 1 mL of culture medium containing radiolabeled SPD alone or radiolabeled SPD in the presence of graduated concentrations of chelator or conjugate. At the end of the incubation period, the tubes were centrifuged; the pellet was washed, digested, and neutralized prior to scintillation counting. Lineweaver-Burk plots indicated simple competitive inhibition with respect to SPD.

The conjugate of spermidine with the monomethyl ester of terephthalic acid, a trication at physiological pH, competed well for the polyamine transporter, having a *K*ᵢ of 3.1 µM. However, when the methyl ester of the amide was cleaved to the carboxylic acid, which is an anion at physiological pH, the *K*ᵢ increased 9-fold to 27 µM. These results indicate that the polyamine cationic centers can bind to negative counter-ions in the transporter. Thus, conjugates bearing cationic centers might show increased binding to the transport apparatus or increased cell internalization when compared to conjugates having anionic characteristics. For example, with the NSPD-(S)-4'-(HO)-DADFT conjugates(Compounds 9 and 10 of FIG. 11B) the *K*ᵢ for the free acid, which exists as the carboxylate anion under the assay conditions, was 73 µM, which is substantially (12.8-fold) higher than that of the corresponding "carboxylate-neutral" ethyl ester, having a *K*ᵢ of 5.7 µM.

### Example 14

### Intracellular Concentrations of Polyamine, Iron Chelators and Polyamine-Chelator Conjugates

During logarithmic growth, cells were treated with the compounds. At the end of the treatment period, cell suspensions were sampled, washed three times in ice-cold, incomplete medium, and pelleted for extraction using 0.6 N perchloric acid, then freeze-fractured in liquid nitrogen/hot water three times. Each supernatant was frozen at -20 °C until analysis of analogue content by HPLC.

*Terephthalic Acid-Polyamine Conjugates* (6 and 7 of FIGS. 11A and 11B). Analytical separation was performed on a Waters Symmetry C₁₈ column (250 mm x 4.6 mm, 5 µm) with a guard column using a Rainin Instrument Company HPLC system. The buffer employed was sodium octanesulfonate (2.5 mM) in potassium phosphate (25 mM), pH 3.0. Mobile phase A consisted of 5% CH₃CN, 95% buffer; mobile phase B consisted of 60% CH₃CN, 40% buffer. The solvent gradient program employed a linear gradient that increased from 20% mobile phase B to 40% mobile phase B over 40 min and a 5-min ramp to 100% B over 5 min. Post-column derivatization used a boric acid buffer system (H₃BO₃, 3.1% w/v; KOH, 2.6% w/v; 2-mercaptoethanol, 0.6% v/v) that contained o-phthaldialdehyde (10 mL of a 4% w/v solution in CH₃OH per L of buffer). The flow rate was 0.4 mL/min, isocratic; UV detection (λₑₓ, 340 nm; λₑₘ, 445 nm) was utilized.

*Norspermidine-(S)-4'-(HO)-DADFT Conjugates* (**9** and **10** of FIG. 11B). Analytical separation was performed on a reversed-phase Supelco Discovery RP Amide C₁₆ column (150 × 4.6 mm, 5 µm) on the system described above, and UV detection was at 300 nm. The mobile phases were pumped at a flow rate of 1.5 mL/min. The buffer and mobile phases A and B were the same as described above. The solvent gradient program employed an initial 10-min isocratic portion with 5% mobile phase B (95% A), followed by a linear gradient increase to 80% mobile phase B at 35 min, a 5-min ramp to 100% B held for 10 min, and ramping back to 5% mobile phase B for 8 min (58-65 min total). This method had a detection limit of 0.2 µM as a direct injection; this corresponds to a tissue concentration of 10 nmol/g wet weight.

In both cases, the concentrations were calculated from the peak area fitted to calibration curves by nonweighted least squares linear regression with Rainin Dynamax HPLC Method Manager software (Rainin Instrument Co.).

Previous studies have shown that DESPM reaches intracellular levels of 400 µM when the extracellular treatment concentration is 30 µM (FIG. 11A). However, the dicationic FDESPM (2), which demonstrated a poor *K*ᵢ for the polyamine transport apparatus, was not effectively concentrated in the cell. Even at an extracellular treatment concentration of 100 µM, FDESPM achieved a cellular level of <20 µM. L1 itself only reached intracellular levels of I µM at an extracellular treatment concentration of 50 µM, whereas its conjugate with spermidine 5 attained an intracellular level of 390 µM at an extracellular concentration of 0.2 µM (FIG. 11A).

Cells treated with methyl terephthalate-SPD conjugate 6 at a concentration of 100 µM achieved an intracellular drug level of 430 µM. However, cells treated with the corresponding acid conjugate (7) at a concentration of 100 µM reached intracellular levels of < 70 µM.

A similar result was observed with the (*S*)-4'-(HO)-DADFT series (**8-10**). In each case, the extracellular treatment concentration was 100 µM. Under these treatment conditions, the intracellular concentration of the parent ligand, (*S*)-4'-(HO)-DADFT (**8**), does not exceed 50 µM (FIG. 11B). However, the ethyl ester of the NSPD-(*S*)-4'-(HO)-DADFT conjugate (**9**) was effectively transported into the cell, achieving a concentration of 947 ± 110 µM; furthermore, the hydrolysis product, acid conjugate **10**, was present at approximately three times the amount of the ester, 2980 ± 280 µM. In cells treated with the NSPD conjugate acid 10 itself, the intracellular drug level rose to about 70 µM. In both series (**6/7, 9/10**), the results are consistent with the significant drop in *K*ᵢ of the esters (**6** and **9**) compared to the corresponding free acid polyamine conjugates (**7** and **10**, respectively), indicating that the transport apparatus prefers substrates that do not carry a negative charge. Furthermore, it seems likely that the esters **6** and **9** were transported into the cells and then hydrolyzed to the respective free acid.

### Example 15

### Iron Clearing Efficiency of Polyamine-Chelator Conjugates

The behavior of the chelator conjugates versus the parent chelator, i.e, the L1 (**5** vs **4** of FIG. 11 A) and (*S*)-4'-(HO)-DADFT series (**9** and **10** vs **8** FIG. 11B), was evaluated in a rodent iron clearance model, the bile duct-cannulated rat. In this model, chelator-promoted iron excretion is measured in both the bile and the urine. Furthermore, because the method allows for collection of multiple bile samples, the kinetics of iron excretion can be determined. In each instance, the drugs were given subcutaneously. L1 and its SPM conjugate (**4** and **5**) were administered at a dose of 450 µmol/kg and (*S*)-4'-(HO)-DADFT (**8**) and its NSPD conjugates (**9** and **10**) were administered at a dose of 300 µmol/kg, i.e., all at equivalent iron-binding capacity. (L1 and its SPM conjugate form 3:1 ligand/metal complexes, whereas the (*S*)-4'-(HO)-DADFT-based compounds form 2:1 ligand/metal complexes. The tissues of animals given the ester **10** contained largely free acid, so it is unlikely that the ethyl ester survives non-specific serum esterases.)

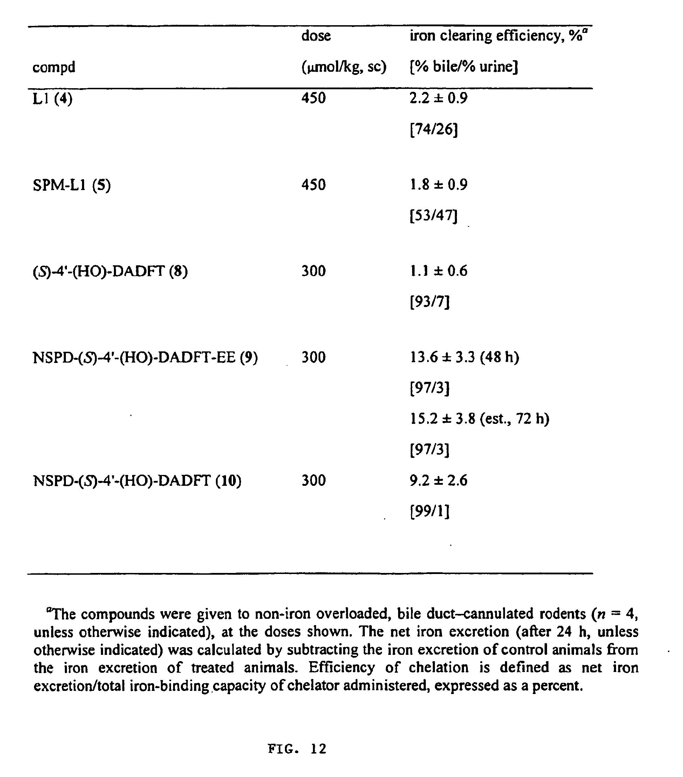

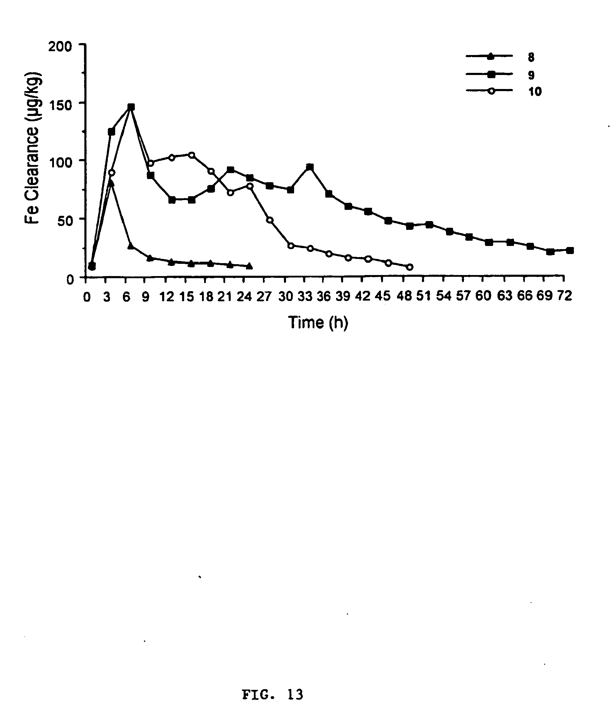

In the rodent model (FIG. 12), L1 (**4**) had an iron-clearing efficiency of 2.2 ± 0.9%; 74% of the iron was in the bile and 26% in the urine. The performance of the corresponding SPM conjugate (**5**) was about the same, 1.8 ± 0.9%, although the mode of iron excretion changed slightly, to 53% biliary and 47% urinary. The efficiency of the parent (*S*)-4'-(HO)-DADFT ligand (**8**) was 1.1 ± 0.6%; the bile/urine ratio was 93:7. Once the NSPD vector was appended to (*S*)-4'-(HO)-DADFT, providing conjugate **10**, the efficiency increased to 9.2 ± 2.6%. In the case of the corresponding ester (**9**), the efficacy increased further, to 13.6 ± 3.3%. The modes of iron excretion were similar to those of the parent compound **8**. However, the profound difference in the ferrokinetics associated with the polyamine-vectored (*S*)-4'-(HO)-DADFTs (**9** and **10**) vs free (*S*)-4'-(HO)-DADFT (**8**) is noteworthy (FIG. 13). Both of the NSPD conjugates (**9** and **10**) elicited protracted biliary iron clearance. In contrast, the unconjugated ligand-induced iron clearance had essentially returned to baseline 9 h after single-dose administration of 8. In the case of the conjugate acid **10**, the return to baseline was not until 48 h after drug administration. In the case of the ester **9**, the iron excretion had not returned to baseline even after 72 h, at which time the animals were euthanized.

### Comparative Example 16

### Synthesis of Terephthalic Acid Conjugates (6 and 7 of FIGS. 11A and 11B)

*N*¹,*N*⁴,*N*⁹-Tris(*tert*-butoxycarbonyl)spermine was acylated in 70% yield with *mono*-methyl terephthalate (**12**), which had been activated with 1,1'-carbonyldiimidazole (CDI). The amine protecting groups of the resulting adduct **13** were removed using methanolic HCl, generating triamino amide-methyl ester **6** in 75% yield. Alternatively, initial cleavage of the ester in intermediate **13** with NaOH in aqueous methanol resulted in tris(BOC) acid **14**. The carbamates of **14** were cleaved with trifluoroacetic acid, followed by ion exchange chromatography, providing the terephthalic acid derivative of spermine as its trihydrochloride salt, **7**, in 45% yield from **13**.

While this invention has been particularly shown and described with references to preferred embodiments thereof, it will be understood by those skilled in the art that various changes in form and details may be made therein without departing from the scope of the invention encompassed by the appended claims.

## Claims

1. A compound which is a conjugate comprising a first moiety which is an iron chelator having an overall formation constant of at least about 10¹² with iron(II) or iron(III) at about 25°C, about pH 7 and about 0.1 M molar strength, and selected from desferrithiocin or an analogue or derivative thereof and a second moiety which is a polyamine, wherein the polyamine moiety includes three or more nitrogen atoms which are capable of being positively charged at physiological pH, or salts, solvates or hydrates thereof.

2. The compound of Claim 1, wherein the iron chelator is 2-(2,4-dihydroxyphenyl)-4(*S*)-methylthiazoline-4-carboxylic acid.

3. A compound represented by Structural Formula (I): 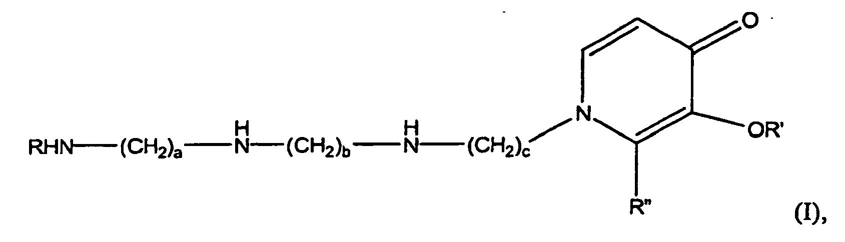 wherein:
a, b and c are each independently integers from 1 to 5;
R is -H or a substituted or unsubstituted alkyl, acyl or aryl group;
R' is -H or a substituted or unsubstituted acyl group; and
R" is -H or a substituted or unsubstituted alkyl group,
or a salt, solvate or hydrate thereof.

4. The compound of Claim 3, wherein R and R' are both -H.

5. The compound of Claim 4, wherein a, b and c are each independently 3 or 4.

6. The compound of Claim 5, wherein a is 3, b is 4 and c is 3.

7. The compound of Claim 5, wherein a, b and c are each 3.

8. A compound represented by Structural Formula (II): 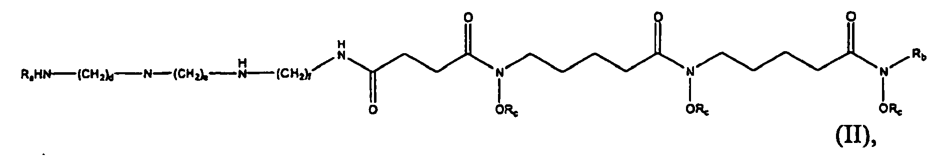 wherein:
d, e and f are each independently integers from 1 to 5;
Rₐ is -H or a substituted or unsubstituted alkyl, acyl or aryl group;
R_{b} is -H or a substituted or unsubstituted alkyl group or a substituted or unsubstituted hydrocarbyl group interrupted by one or more oxygen atoms; and
each R_{c} is independently -H or a substituted or unsubstituted acyl group,
or a salt, solvate or hydrate thereof.

9. The compound of Claim 8, wherein Rₐ and each R_{c} are -H.

10. The compound of Claim 9, wherein d, e and f are each independently 3 or 4.

11. The compound of Claim 10, wherein R_{b} is an unsubstituted hydrocarbyl group interrupted by one or more oxygen atoms.

12. The compound of Claim 10, wherein d is 3, e is 4 and f is 3.

13. The compound of Claim 12, wherein R_{b} is a 3,6,9-trioxadecyl group.

14. The compound of Claim 10, wherein d, e and f are each 3.

15. A compound represented by Structural Formula (III): 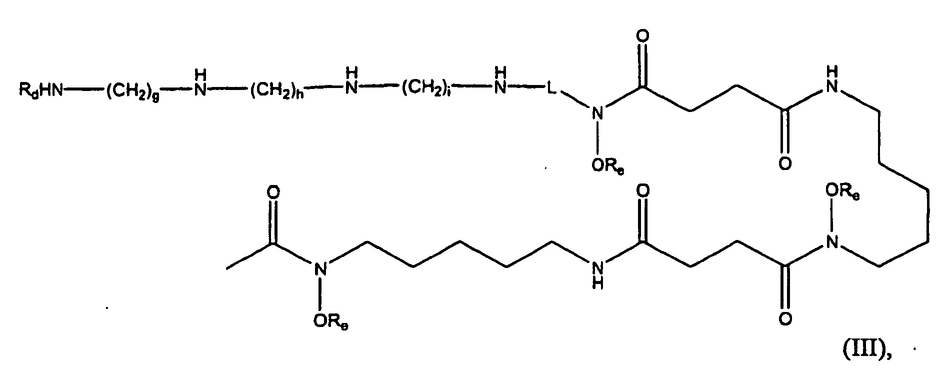 wherein:
g, h and i are each independently an integer from 1 to 5;
L is a linking group;
R_{d} is -H or a substituted or unsubstituted alkyl, acyl or aryl group; and
each Rₑ is independently -H or a substituted or unsubstituted acyl group,
or a salt, solvate or hydrate thereof.

16. The compound of Claim 15, wherein R_{d} and each Rₑ are -H.

17. The compound of Claim 16, wherein g, h and i are each independently 3 or 4.

18. The compound of Claim 17, wherein L is a substituted or unsubstituted alkylene group.

19. The compound of Claim 18, wherein g, h and i are each 3.

20. The compound of Claim 19, wherein L is a pentamethylene group.

21. The compound of Claim 18, wherein g is 3, h is 4 and i is 3.

22. A pharmaceutical composition comprising a pharmaceutically acceptable carrier or diluent and a compound as defined in any one of Claims 1 to 21.

23. Use of a pharmaceutical composition comprising a pharmaceutically acceptable carrier or diluent and a compound as defined in any one of Claims 1 to 21 or a compound as defined in any one of Claims 1 to 21 in the manufacture of a medicament for treating cancer or a tumor in a subject.

24. The use of Claim 23, comprising one or more polyamine synthesis inhibitors for administration to the subject.

25. The use of Claim 24, wherein the polyamine synthesis inhibitor is an ornithine decarboxylase inhibitor, an S-adenosylmethionine decarboxylase inhibitor, a spermidine synthase inhibitor or a spermine synthase inhibitor.

26. Use of a pharmaceutical composition comprising a pharmaceutically acceptable carrier or diluent and a compound as defined in any one of Claims 1 to 21, or a compound as defined in any one of Claims 1 to 21, in the manufacture of a medicament for treating a subject suffering from a metal overload condition.

27. The use of Claim 26, wherein the metal overload condition is an iron overload condition.

28. The use of Claim 27, wherein the iron overload condition is **characterized by** global iron overload.

29. The use of Claim 28, wherein the iron overload condition is primary hemochromatosis.

30. The use of Claim 28, wherein the iron overload condition is caused by chronic blood transfusion.

31. The use of Claim 27, wherein the iron overload condition is **characterized by** focal iron overload.

32. The use of Claim 31, wherein the iron overload condition is **characterized by** a neurological or a neurodegenerative disorder.

33. Use of a compound as defined in any one of Claims 1 to 21 or a pharmaceutical composition comprising a pharmaceutically acceptable carrier or diluent and a compound as defined in any one of Claims 1 to 21, in the manufacture of a medicament for increasing uptake of a metal chelator into a cell.

34. A pharmaceutical composition comprising a pharmaceutically acceptable carrier or diluent and a compound as defined in any one of Claims 1 to 21, or a compound as defined in any one of Claims 1 to 21, for treating cancer or a tumor in a subject.

35. The composition or compound of Claim 34, for administration to the subject with one or more polyamine synthesis inhibitors.

36. The composition or compound of Claim 35, wherein the polyamine synthesis inhibitor is an ornithine decarboxylase inhibitor, an S-adenosylmethionine decarboxylase inhibitor, a spermidine synthase inhibitor or a spermine synthase inhibitor.

37. A pharmaceutical composition comprising a pharmaceutically acceptable carrier or diluent and a compound as defined in any one of Claims 1 to 21, or a compound as defined in any one of Claims 1 to 21, for treating a subject suffering from a metal overload condition.

38. The composition or compound of Claim 37, wherein the metal overload condition is an iron overload condition.

39. The composition or compound of Claim 38, wherein the iron overload condition is **characterized by** global iron overload.

40. The composition or compound of Claim 39, wherein the iron overload condition is primary hemochromatosis.

41. The composition or compound of Claim 39, wherein the iron overload condition is caused by chronic blood transfusion.

42. The composition or compound of Claim 38, wherein the iron overload condition is **characterized by** focal iron overload.

43. The composition or compound of Claim 42, wherein the iron overload condition is **characterized by** a neurological or a neurodegenerative disorder.

44. A compound as defined in any one of Claims 1 to 21 or a pharmaceutical composition comprising a pharmaceutically acceptable carrier or diluent and a compound as defined in any one of Claims 1 to 21, for increasing uptake of a metal chelator into a cell.

45. A pharmaceutical composition comprising a pharmaceutically acceptable carrier ox diluent and a compound as defined in any one of Claims 1 to 21, or a compound as defined in any one of Claims 1 to 21, for use in medical therapy.

## Patentansprüche

1. Verbindung, bei der es sich um ein Konjugat handelt, das eine erste Einheit, die ein Eisen-Chelatbildner mit einer Gesamtbildungskonstante von mindestens etwa 10¹² mit Eisen(II) oder Eisen(III) bei etwa 25 °C, einem pH-Wert von etwa 7 und einer Ionenstärke von etwa 0,1 M ist und aus Desferrithiocin oder einem Analogon oder Derivat desselben ausgewählt ist, und eine zweite Einheit, die ein Polyamin ist, wobei die Polyamineinheit 3 oder mehr Stickstoffatome umfasst, die bei physiologischem pH-Wert positiv geladen sein können, umfasst, oder Salze, Solvate oder Hydrate derselben.

2. Verbindung nach Anspruch 1, wobei der Eisen-Chelatbildner 2-(2,4-Dihydroxyphenyl)-4(S)-methylthiazolin-4-carbonsäure ist.

3. Verbindung der Strukturformel (I): 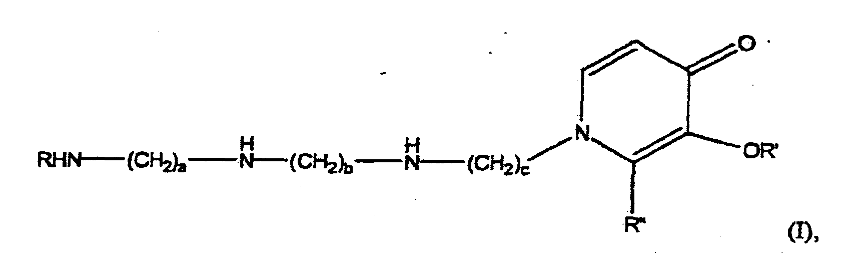 worin:
a, b und c jeweils unabhängig voneinander ganze Zahlen von 1 bis 5 sind;
R für -H oder eine substituierte oder unsubstituierte Alkyl-, Acyl- oder Arylgruppe steht;
R' für -H oder eine substituierte oder unsubstituierte Acylgruppe steht; und
R" für -H oder eine substituierte oder unsubstituierte Alkylgruppe steht,
oder ein Salz, Solvat oder Hydrat derselben.

4. Verbindung nach Anspruch 3, wobei R und R' beide für -H stehen.

5. Verbindung nach Anspruch 4, wobei a, b und c jeweils unabhängig voneinander 3 oder 4 sind.

6. Verbindung nach Anspruch 5, wobei a 3 ist, b 4 ist und c 3 ist.

7. Verbindung nach Anspruch 5, wobei a, b und c jeweils 3 sind.

8. Verbindung der Strukturformel (II): 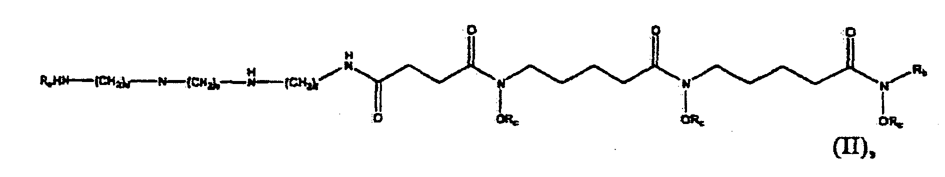 worin:
d, e und f jeweils unabhängig voneinander ganze Zahlen von 1 bis 5 sind;
Rₐ für -H oder eine substituierte oder unsubstituierte Alkyl-, Acyl- oder Arylgruppe steht;
R_{b} für -H oder eine substituierte oder unsubstituierte Alkylgruppe oder eine substituierte oder unsubstituierte Hydrocarbylgruppe, die von einem oder mehreren Sauerstoffatomen unterbrochen ist, steht; und
jedes R_{c} unabhängig voneinander für -H oder eine substituierte oder unsubstituierte Acylgruppe steht,
oder ein Salz, Solvat oder Hydrat derselben.

9. Verbindung nach Anspruch 8, wobei Rₐ und jedes R_{c} für -H stehen.

10. Verbindung nach Anspruch 9, wobei d, e und f jeweils unabhängig voneinander 3 oder 4 sind.

11. Verbindung nach Anspruch 10, wobei R_{b} für eine unsubstituierte Hydrocarbylgruppe, die von einem oder mehreren Sauerstoffatomen unterbrochen ist, steht.

12. Verbindung nach Anspruch 10, wobei d 3 ist, e 4 ist und f 3 ist.

13. Verbindung nach Anspruch 12, wobei R_{b} für eine 3,6,9-Trioxadecylgruppe steht.

14. Verbindung nach Anspruch 10, wobei d, e und f jeweils 3 sind.

15. Verbindung der Strukturformel (III): 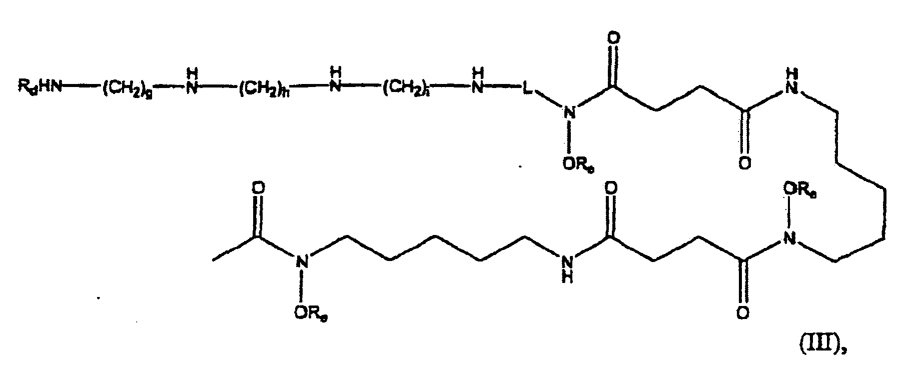 worin:
g, h und i jeweils unabhängig voneinander eine ganze Zahl von 1 bis 5 sind;
L für eine Verknüpfungsgruppe steht;
R_{d} für -H oder eine substituierte oder unsubstituierte Alkyl-, Acyl- oder Arylgruppe steht; und
jedes Rₑ unabhängig voneinander für -H oder eine substituierte oder unsubstituierte Acylgruppe steht,
oder ein Salz, Solvat oder Hydrat derselben.

16. Verbindung nach Anspruch 15, wobei R_{d} und jedes Rₑ für -H stehen.

17. Verbindung nach Anspruch 16, wobei g, h und i jeweils unabhängig voneinander 3 oder 4 sind.

18. Verbindung nach Anspruch 17, wobei L für eine substituierte oder unsubstituierte Alkylengruppe steht.

19. Verbindung nach Anspruch 18, wobei g, h und i jeweils 3 sind.

20. Verbindung nach Anspruch 19, wobei L für eine Pentamethylengruppe steht.

21. Verbindung nach Anspruch 18, wobei g 3 ist, h 4 ist und i 3 ist.

22. Pharmazeutische Zusammensetzung, die einen pharmazeutisch akzeptablen Träger oder ein pharmazeutisch akzeptables Verdünnungsmittel und eine Verbindung gemäß der Definition in einem der Ansprüche 1 bis 21 umfasst.

23. Verwendung einer pharmazeutischen Zusammensetzung, die einen pharmazeutisch akzeptablen Träger oder ein pharmazeutisch akzeptables Verdünnungsmittel und eine Verbindung gemäß der Definition in einem der Ansprüche 1 bis 21 oder eine Verbindung gemäß der Definition in einem der Ansprüche 1 bis 21 umfasst, bei der Herstellung eines Medikaments zur Behandlung von Krebs oder einem Tumor bei einem Subjekt.

24. Verwendung nach Anspruch 23, wobei ein oder mehrere Polyaminsyntheseinhibitoren zur Verabreichung an das Subjekt umfasst werden.

25. Verwendung nach Anspruch 24, wobei der Polyaminsyntheseinhibitor ein Ornithindecarboxylaseinhibitor, ein S-Adenosylmethionindecarboxylaseinhibitor, ein Spermidinsynthaseinhibitor oder ein Sperminsynthaseinhibitor ist.

26. Verwendung einer pharmazeutischen Zusammensetzung, die einen pharmazeutisch akzeptablen Träger oder ein pharmazeutisch akzeptables Verdünnungsmittel und eine Verbindung gemäß der Definition in einem der Ansprüche 1 bis 21 oder eine Verbindung gemäß der Definition in einem der Ansprüche 1 bis 21 umfasst, bei der Herstellung eines Medikaments zur Behandlung eines Subjekts, das an einem Metallüberladungszustand leidet.

27. Verwendung nach Anspruch 26, wobei der Metallüberladungszustand ein Eisenüberladungszustand ist.

28. Verwendung nach Anspruch 27, wobei der Eisenüberladungszustand durch eine globale Eisenüberladung **gekennzeichnet** ist.

29. Verwendung nach Anspruch 28, wobei der Eisenüberladungszustand primäre Hämochromatose ist.

30. Verwendung nach Anspruch 28, wobei der Eisenüberladungszustand durch chronische Bluttransfusion verursacht ist.

31. Verwendung nach Anspruch 27, wobei der Eisenüberladungszustand durch eine fokale Eisenüberladung **gekennzeichnet** ist.

32. Verwendung nach Anspruch 31, wobei der Eisenüberladungszustand durch eine neurologische oder neurodegenerative Störung **gekennzeichnet** ist.

33. Verwendung einer Verbindung gemäß der Definition in einem der Ansprüche 1 bis 21 oder einer pharmazeutischen Zusammensetzung, die einen pharmazeutisch akzeptablen Träger oder ein pharmazeutisch akzeptables Verdünnungsmittel und eine Verbindung gemäß der Definition in einem der Ansprüche 1 bis 21 umfasst, bei der Herstellung eines Medikaments zum Erhöhen der Aufnahme eines Metall-Chelatbildners in eine Zelle.

34. Pharmazeutische Zusammensetzung, die einen pharmazeutisch akzeptablen Träger oder ein pharmazeutisch akzeptables Verdünnungsmittel und eine Verbindung gemäß der Definition in einem der Ansprüche 1 bis 21 umfasst, oder eine Verbindung gemäß der Definition in einem der Ansprüche 1 bis 21 zur Behandlung von Krebs oder einem Tumor bei einem Subjekt.

35. Zusammensetzung oder Verbindung nach Anspruch 34 zur Verabreichung an das Subjekt mit einem oder mehreren Polyaminsyntheseinhibitoren.

36. Zusammensetzung oder Verbindung nach Anspruch 35, wobei der Polyaminsyntheseinhibitor ein Ornithindecarboxylaseinhibitor, ein S-Adenosylmethionindecarboxylaseinhibitor, ein Spermidinsynthaseinhibitor oder ein Sperminsynthaseinhibitor ist.

37. Pharmazeutische Zusammensetzung, die einen pharmazeutisch akzeptablen Träger oder ein pharmazeutisch akzeptables Verdünnungsmittel und eine Verbindung gemäß der Definition in einem der Ansprüche 1 bis 21 umfasst, oder eine Verbindung gemäß der Definition in einem der Ansprüche 1 bis 21 zur Ansprüche 1 bis 21 zur Behandlung eines Subjekts, das an einem Metallüberladungszustand leidet.

38. Zusammensetzung oder Verbindung nach Anspruch 37, wobei der Metallüberladungszustand ein Eisenüberladungszustand ist.

39. Zusammensetzung oder Verbindung nach Anspruch 38, wobei der Eisenüberladungszustand durch eine globale Eisenüberladung **gekennzeichnet** ist.

40. Zusammensetzung oder Verbindung nach Anspruch 39, wobei der Eisenüberladungszustand primäre Hämochromatose ist.

41. Zusammensetzung oder Verbindung nach Anspruch 39, wobei der Eisenüberladungszustand durch chronische Bluttransfusion verursacht ist.

42. Zusammensetzung oder Verbindung nach Anspruch 38, wobei der Eisenüberladungszustand durch eine fokale Eisenüberladung **gekennzeichnet** ist.

43. Zusammensetzung oder Verbindung nach Anspruch 42, wobei der Eisenüberladungszustand durch eine neurologische oder neurodegenerative Störung **gekennzeichnet** ist.

44. Verbindung gemäß der Definition in einem der Ansprüche 1 bis 21 oder pharmazeutische Zusammensetzung, die einen pharmazeutisch akzeptablen Träger oder ein pharmazeutisch akzeptables Verdünnungsmittel und eine Verbindung gemäß der Definition in einem der Ansprüche 1 bis 21 umfasst, zum Erhöhen der Aufnahme eines Metall-Chelatbildners in eine Zelle.

45. Pharmazeutische Zusammensetzung, die einen pharmazeutisch akzeptablen Träger oder ein pharmazeutisch akzeptables Verdünnungsmittel und eine zeptables Verdünnungsmittel und eine Verbindung gemäß der Definition in einem der Ansprüche 1 bis 21 umfasst, oder Verbindung gemäß der Definition in einem der Ansprüche 1 bis 21 zur Verwendung in der medizinischen Therapie.

## Revendications

1. Composé qui est un conjugué comprenant un premier groupe qui est un chélateur du fer possédant une constante globale de formation d'au moins environ 10¹² avec du fer (II) ou du fer (III) à environ 25 °C, à un pH d'environ 7, à une concentration molaire d'environ 0,1 M, et choisi parmi la desferrithiocine ou un analogue ou un dérivé de celle-ci et un second groupe qui est une polyamine, dans lequel le groupe de polyamine comprend trois atomes d'azote ou plus qui peuvent être chargés positivement à pH physiologique, ou ses sels, solvates ou hydrates.

2. Composé selon la revendication 1, dans lequel le chélateur du fer est l'acide 2-(2,4-dihydroxy-phényl)-4(S)-méthylthiazoline-4-carboxylique.

3. Composé représenté par la formule structurale (I) : 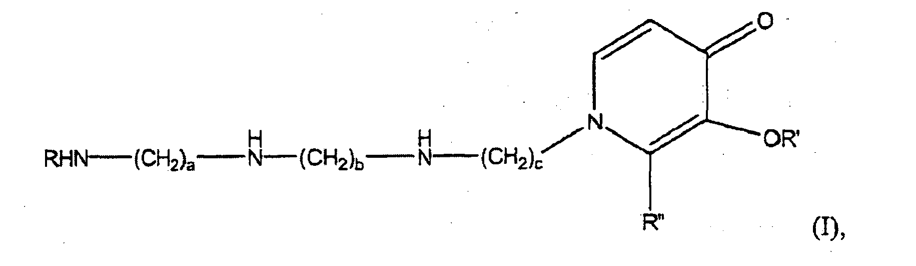 dans laquelle :
a, b et c sont chacun indépendamment des nombres entiers allant de 1 à 5 ;
R est -H ou un groupe alkyle, acyle ou aryle substitué ou non substitué ;
R' est -H ou un groupe acyle substitué ou non substitué ; et
R" est -H ou un groupe alkyle substitué ou non substitué,
ou un sel, un solvate ou un hydrate de celui-ci.

4. Composé selon la revendication 3, dans lequel R et R' sont tous les deux -H.

5. Composé selon la revendication 4, dans lequel a, b et c valent chacun indépendamment 3 ou 4.

6. Composé selon la revendication 5, dans lequel a vaut 3, b vaut 4 et c vaut 3.

7. Composé selon la revendication 5, dans lequel a, b et c valent chacun 3.

8. Composé représenté par la formule structurale (II) : 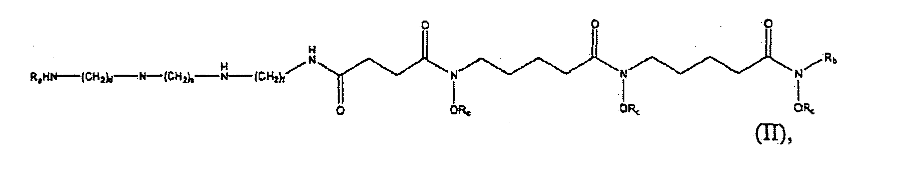 dans laquelle :
d, e et f sont chacun indépendamment des nombres entiers allant de 1 à 5 ;
Rₐ est -H ou un groupe alkyle, acyle ou aryle substitué ou non substitué ;
R_{b} est -H ou un groupe alkyle substitué ou non substitué ou un groupe hydrocarbyle substitué ou non substitué interrompu par un ou plusieurs atomes d'oxygène ; et
chaque R_{c} est indépendamment -H ou un groupe acyle substitué ou non substitué,
ou un sel, un solvate ou un hydrate de celui-ci.

9. Composé selon la revendication 8, dans lequel Rₐ et chaque R_{c} sont -H.

10. Composé selon la revendication 9, dans lequel d, e et f valent chacun indépendamment 3 ou 4.

11. Composé selon la revendication 10, dans lequel R_{b} est un groupe hydrocarbyle non substitué interrompu par un ou plusieurs atomes d'oxygène.

12. Composé selon la revendication 10, dans lequel d vaut 3, e vaut 4 et f vaut 3.

13. Composé selon la revendication 12, dans lequel R_{b} est un groupe 3,6,9-trioxadécyle.

14. Composé selon la revendication 10, dans lequel d, e et f valent chacun 3.

15. Composé représenté par la formule structurale (III) : 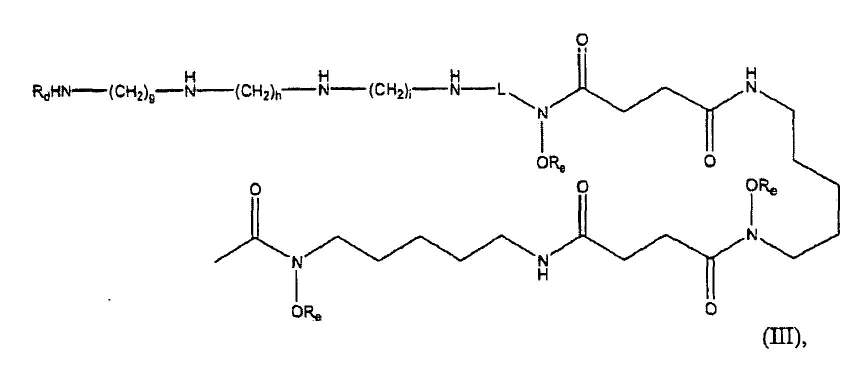 dans laquelle :
g, h et i sont chacun indépendamment un nombre entier allant de 1 à 5 ;
L est un groupe de liaison ;
R_{d} est -H ou un groupe alkyle, acyle ou aryle substitué ou non substitué ; et chaque Rₑ est indépendamment -H ou un groupe acyle substitué ou non substitué,
ou un sel, un solvate ou un hydrate de celui-ci.

16. Composé selon la revendication 15, dans lequel R_{d} et chaque Rₑ sont -H.

17. Composé selon la revendication 16, dans lequel g, h et i valent chacun indépendamment 3 ou 4.

18. Composé selon la revendication 17, dans lequel L est un groupe alkylène substitué ou non substitué.

19. Composé selon la revendication 18, dans lequel g, h et i valent chacun 3.

20. Composé selon la revendication 19, dans lequel L est un groupe pentaméthylène.

21. Composé selon la revendication 18, dans lequel g vaut 3, h vaut 4 et i vaut 3.

22. Composition pharmaceutique comprenant un support ou un diluant pharmaceutiquement acceptable et un composé tel que défini dans l'une quelconque des revendications 1 à 21.

23. Utilisation d'une composition pharmaceutique comprenant un support ou un diluant pharmaceutiquement acceptable et un composé tel que défini dans l'une quelconque des revendications 1 à 21 ou d'un composé tel que défini dans l'une quelconque des revendications 1 à 21 pour la préparation d'un médicament destiné au traitement du cancer ou d'une tumeur chez un sujet.

24. Utilisation selon la revendication 23, comprenant un ou plusieurs inhibiteurs de la synthèse de polyamines pour l'administration au sujet.

25. Utilisation selon la revendication 24, dans laquelle l'inhibiteur de la synthèse de polyamines est un inhibiteur de l'ornithine décarboxylase, un inhibiteur de la (S)-adénosylméthionine décarboxylase, un inhibiteur de la spermidine synthase ou un inhibiteur de la spermine synthase.

26. Utilisation d'une composition pharmaceutique comprenant un support ou un diluant pharmaceutiquement acceptable et un composé tel que défini dans l'une quelconque des revendications 1 à 21, ou d'un composé tel que défini dans l'une quelconque des revendications 1 à 21, pour la préparation d'un médicament destiné au traitement d'un sujet souffrant d'un état de surcharge en métal.

27. Utilisation selon la revendication 26, dans laquelle l'état de surcharge en métal est un état de surcharge en fer.

28. Utilisation selon la revendication 27, dans laquelle l'état de surcharge en fer est **caractérisé par** une surcharge globale en fer.

29. Utilisation selon la revendication 28, dans laquelle l'état de surcharge en fer est une hémochromatose primaire.

30. Utilisation selon la revendication 28, dans laquelle l'état de surcharge en fer est provoqué par une transfusion sanguine chronique.

31. Utilisation selon la revendication 27, dans laquelle l'état de surcharge en fer est **caractérisé par** une surcharge focale en fer.

32. Utilisation selon la revendication 31, dans laquelle l'état de surcharge en fer est **caractérisé par** un trouble neurologique ou neurodégénératif.

33. Utilisation d'un composé tel que défini dans l'une quelconque des revendications 1 à 21 ou d'une composition pharmaceutique comprenant un support ou un diluant pharmaceutiquement acceptables et un composé tel que défini dans l'une quelconque des revendications 1 à 21, pour la préparation d'un médicament destiné à accroître le recaptage d'un chélateur de métal dans une cellule.

34. Composition pharmaceutique comprenant un support ou un diluant pharmaceutiquement acceptable et un composé tel que défini dans l'une quelconque des revendications 1 à 21 ou un composé tel que défini dans l'une quelconque des revendications 1 à 21 pour traiter un cancer ou une tumeur chez un sujet.

35. Composition ou composé selon la revendication 34, pour l'administration au sujet avec un ou plusieurs inhibiteurs de la synthèse de polyamines.

36. Composition ou composé selon la revendication 35, dans laquelle/lequel l'inhibiteur de la synthèse de polyamines est un inhibiteur de l'ornithine décarboxylase, un inhibiteur de la (S)-adénosylméthionine décarboxylase, un inhibiteur de la spermidine synthase ou un inhibiteur de la spermine synthase.

37. Composition pharmaceutique comprenant un support ou un diluant pharmaceutiquement acceptable et un composé tel que défini dans l'une quelconque des revendications 1 à 21 ou un composé tel que défini dans l'une quelconque des revendications 1 à 21 pour traiter un sujet souffrant d'un état de surcharge en métal.

38. Composition ou composé selon la revendication 37, dans laquelle/lequel l'état de surcharge en métal est un état de surcharge en fer.

39. Composition ou composé selon la revendication 38, dans laquelle/lequel l'état de surcharge en fer est **caractérisé par** une surcharge globale en fer.

40. Composition ou composé selon la revendication 39, dans laquelle/lequel l'état de surcharge en fer est une hémochromatose primaire.

41. Composition ou composé selon la revendication 39, dans laquelle/lequel l'état de surcharge en fer est provoqué par une transfusion sanguine chronique.

42. Composition ou composé selon la revendication 38, dans laquelle/lequel l'état de surcharge en fer est **caractérisé par** une surcharge focale en fer.

43. Composition ou composé selon la revendication 42, dans laquelle/lequel l'état de surcharge en fer est **caractérisé par** un trouble neurologique ou neurodégénératif.

44. Composé tel que défini dans l'une quelconque des revendications 1 à 21 ou composition pharmaceutique comprenant un support ou un diluant pharmaceutiquement acceptable et un composé tel que défini dans l'une quelconque des revendications 1 à 21, pour accroître le recaptage d'un chélateur de métal dans une cellule.

45. Composition pharmaceutique comprenant un support ou un diluant pharmaceutiquement acceptable et un composé tel que défini dans l'une quelconque des revendications 1 à 21, ou un composé tel que défini dans l'une quelconque des revendications 1 à 21, pour une utilisation en thérapie médicale.
